# EUROPÄISCHE PATENTSCHRIFT

(11) **EP 1 937 174 B1**
(45) Veröffentlichungstag und Bekanntmachung des Hinweises auf die Patenterteilung: **19.09.2012**
(21) Anmeldenummer: 06805862.7
(22) Anmeldetag: 26.09.2006
(51) Int. Cl.: A61B 18/14, A61B 17/28, A61B 17/29

(54) **ELEKTROCHIRURGISCHES INSTRUMENT**
ELECTROSURGICAL INSTRUMENT
INSTRUMENT ELECTROCHIRURGICAL

(30) Priorität: 04.10.2005 DE 102005047405; 13.09.2006 DE 102006042985
(43) Veröffentlichungstag der Anmeldung: 02.07.2008
(73) Patentinhaber: ERBE Elektromedizin GmbH, 72072 Tübingen (DE)
(72) Erfinder: HAFNER, Dieter, 72072 Tübingen (DE); EISELE, Florian, 72074 Tübingen (DE)
(74) Vertreter: Bohnenberger, Johannes
(86) Internationale Anmeldenummer: PCT/EP2006/009325
(87) Internationale Veröffentlichungsnummer: WO 2007/039185

(56) Entgegenhaltungen:
- DE-A1- 1 919 485
- DE-A1- 2 019 891
- DE-A1- 4 416 499
- GB-A- 2 156 222
- US-A- 3 911 241
- US-A- 4 274 413
- US-A- 4 311 145
- US-A1- 2004 143 263

## Beschreibung

Die Erfindung betrifft ein elektrochirurgisches Instrument nach Patentanspruch 1 und Patentanspruch 9

Elektrochirurgische Instrumente , wie zum Beispiel aus der US3911241 bekannt, werden seit vielen Jahren in der Hochfrequenz-Chirurgie eingesetzt, um biologisches Gewebe zu koagulieren oder auch zu schneiden. Bei einer Koagulation wird ein hochfrequenter Strom durch das zu behandelnde Gewebe geleitet, so dass sich dieses aufgrund Eiweißgerinnung und Dehydratation verändert. Das Gewebe zieht sich dabei derart zusammen, dass die Gefäße verschlossen und Blutungen gestillt werden. Nach erfolgter Koagulation ist das Gewebe unter Vermeidung starker Blutungen vollständig durchtrennbar, sowohl mit Hilfe von hochfrequentem Strom, als auch auf mechanischem Wege.

Elektrochirurgische Vorgänge sind sowohl monopolar als auch bipolar durchführbar. Bei der monopolaren Technik führt der Strompfad üblicherweise vom elektrochirurgischen Instrument über das zu behandelnde Gewebe zu einer Neutralelektrode und von dieser zurück zu einem HF-Generator. Immer mehr an Bedeutung gewinnen jedoch bipolare Instrumente, die mit zwei voneinander elektrisch isolierten Abschnitten ausgebildet sind. Der Stromweg zwischen den Elektrodenteilen ist damit kalkulierbar und verläuft nicht weite Strecken durch den Körper des Patienten. Damit reduziert sich die Beeinflussung von beispielsweise Herzschrittmachern oder sonstigen Geräten, die während der Operation an dem Patienten angeschlossen sind.

Bipolare Instrumente weisen meist zwei gelenkig miteinander verbundene Branchen auf, an deren proximalen Enden Griffeinrichtungen zur Handhabung der Branchen vorgesehen sind. An distalen Enden der Branchen befinden sich Elektrodenteile zum Fassen von Gewebe und zum Durchleiten des hochfrequenten Stromes durch das Gewebe. Der von einem HF-Generator gelieferte HF-Strom wird dazu über Strömzuführungseinrichtungen zu den Elektrodenteilen des bipolaren Instruments geleitet.

Ähnlich aufgebaut sind auch laparoskopische Instrumente, also Rohrschaftinstrumente, wobei hier die Bewegung der Branchen über einen im Inneren des Rohrschaftes angeordneten Umlenkmechanismus auf die an einem distalen Ende des Rohrschaftes ausgebildeten Elektrodenteile übertragen wird. Die Branchen werden also über den Umlenkmechanismus derart verlängert, dass die Elektrodenteile über den Rohrschaft in Körperhöhlen (z. B. die Bauchhöhle) einführbar sind und von "außerhalb" betätigt werden können. Das bedeutet, dass die proximalen Enden der Branchen bzw. der proximale Bereich der Branchen über den Umlenkmechanismus in die distalen Enden bzw. den distalen Bereich der Branchen mit den Elektrodenteilen überführt werden.

Monopolare Instrumente können ebenfalls für die offene Chirurgie, wie auch für minimal-invasive Eingriffe (in der Regel Endoskope) vorgesehen werden.

Um eine sichere Thermofusion biologischen Gewebes zu erzielen, sind verschiedene Kriterien zu beachten. So muss das Gewebe, z. B. ein Blutgefäß, im Falle bipolarer Instrumente sicher zwischen den Elektrodenteilen arretierbar sein, um ein Abgleiten des Gewebes zu vermeiden. Dazu ist es erforderlich, dass über die Elektrodenteile ein bestimmter Druck auf das Gewebe ausgeübt wird. Ferner ist darauf zu achten, dass die Elektrodenteile nur bis zu einem definierten Mindestabstand zusammengeführt werden können, um einen unerwünschten Kurzschluss zwischen den Elektrodenteilen zu vermeiden.

Um nun eine Koagulation oder auch einen Schneidvorgang durchführen zu können, sind die bekannten Instrumente mit Schaltern, wie z. B. Hand- oder Fußschalter, verbunden. Über diese lässt sich der hochfrequente Strom aktivieren, der dann über die Elektrodenteile an das zu behandelnde Gewebe herangeführt wird.

Bei oben beschriebenen Instrumenten können durch eine versehentliche Betätigung der Schalter Koagulationen und/oder Schneidvorgänge zu ungünstigen Zeitpunkten ausgelöst werden. So sind die Branchen oftmals nicht vollständig geschlossen und das Gewebe ist nicht sachgemäß zwischen den Branchen arretiert, obgleich bereits eine Stromzufuhr an das Gewebe stattfindet. Dies hat z. B. unvollständige Thermofusionen zur Folge, was zu gefährlichen Nachblutungen oder zu Koagulationen an unerwünschten Stellen führen kann. Auch bei monopolaren Instrumenten kann eine Stromzufuhr zu einem ungünstigen Zeitpunkt Schäden an dem zu behandelnden Gewebe bewirken.

Zudem benötigen Hand- oder Fußschalter zusätzlichen Bauraum und gestalten das Instrument und damit das gesamte Operationsfeld unübersichtlich.

Der Erfindung liegt daher die Aufgabe zu Grunde, ein elektrochirurgisches Instrument der eingangs genannten Art dahin gehend aufzuzeigen, dass es einfach und kostengünstig herstellbar ist, wobei mit dem Instrument ein chirurgischer Eingriff einfach und sicher durchführbar sein soll.

Diese Aufgabe wird durch ein elektrochirurgisches Instrument nach Patentanspruch 1 gelöst.

Insbesondere wird die Aufgabe durch ein elektrochirurgisches Instrument zum Koagulieren und/oder Schneiden biologischen Gewebes gelöst, das Folgendes umfasst:
- zwei gegeneinander bewegbar verbundene Branchen,
- Griffeinrichtungen an einem proximalen Bereich der Branchen bzw. des Instruments zum Zusammenführen der Branchen,
- Elektrodenteile an einem distalen Bereich der Branchen bzw. des Instruments zum Fassen von Gewebe und zum Durchleiten eines HF-Stromes durch das Gewebe,
- Stromzuführungseinrichtungen zum Zuführen des HF-Stromes zu den Elektrodenteilen von einem HF-Generator,
- eine Schalteinrichtung zum Aktivieren des HF-Stromes bei zusammengeführten Branchen,
- mindestens ein Beabstandungselement zur Ausbildung eines definierten Minimalabstandes zwischen den Elektrodenteilen,
- mindestens ein elastisch verformbares Element, das derart an mindestens einer der Branchen oder den Griffeinrichtungen angeordnet ist, dass beim Schließen der Branchen und Erreichen des Minimalabstandes mindestens ein Bereich der Griffeinrichtungen im proximalen Bereich zur Betätigung der Schalteinrichtung weiter bewegbar ist.

Ein wesentlicher Punkt der Erfindung liegt darin, dass Beabstandungselement, elastisch verformbares Element und Schalteinrichtung derart ausgebildet und in Bezug zueinander angeordnet sind, dass die der Schalteinrichtung gegenüberliegende Branche oder Griffeinrichtung in Kontakt mit der Schalteinrichtung zu deren Betätigung gebracht werden kann. Dabei durchläuft der mindestens eine Bereich der Griffeinrichtungen im proximalen Bereich beim Zusammenführen verschiedene "Wegbereiche". Das Durchlaufen eines ersten "Wegbereichs" dient der Bewegung der Elektrodenteile zum Fassen und Arretieren des Gewebes, wobei ein bestimmter Minimalabstand zwischen den Elektrodenteilen aufgrund des am Instrument vorgesehenen Beabstandungselements nicht unterschritten werden kann. Auf diese Weise lässt sich ein Kurzschluss zwischen den Elektrodenteilen vermeiden. Sobald nun die Griffeinrichtungen bzw. die Branchen derart zusammengeführt sind, dass der Minimalabstand erreicht und das zu behandelnde Gewebe sicher arretiert ist, kann mindestens ein Bereich der Griffeinrichtungen im proximalen Bereich, ggf. auch mindestens eine Branche aufgrund des elastisch verformbaren Elements derart weiter bewegt werden, dass die Betätigung der ebenfalls im proximalen Bereich an dieser oder der gegenüberliegenden Branche oder Griffeinrichtung ausgebildeten Schalteinrichtung ermöglicht wird. Mindestens die weiterbewegbare Griffeinrichtung bzw. Branche durchläuft dabei einen zweiten "Wegbereich", um Schalteinrichtung und gegenüberliegende Branche - wie oben angegeben - miteinander in Kontakt zu bringen. Das heißt, die Branchen bzw. Griffeinrichtungen lassen sich in ihrem proximalen Bereich noch weiter zusammenführen, auch wenn eine Bewegung der Elektrodenteile hier nicht mehr vorgesehen ist. Wichtig hierbei ist, dass das elastisch verformbare Element in den Kraftübertragungsweg integriert ist und so beide Wegbereiche durchlaufen werden können. Über die Schalteinrichtung lässt sich die Stromzufuhr von dem HF-Generator hin zu den Elektrodenteilen aktivieren. Die Stromzuführungseinrichtungen weisen hierzu zumindest ein Stromanschlusselement am Instrument auf und z. B. Leitungen, die durch das Instrument bis hin zu den Elektrodenteilen verlaufen, um eine Stromeinspeisung in das Gewebe über die Elektrodenteile zu gewährleisten. Das Zusammenführen der Branchen bzw. Griffeinrichtungen überführt die Elektrodenteile im Prinzip von einer Ruheposition (geöffnetes Maulteil) in eine Arbeitsposition (im Wesentlichen geschlossenes Maulteil) zum Fassen von Gewebe.

Mit dieser Anordnung kann auf aufwändige Hand- oder Fußschalter zur Aktivierung des HF-Stromes verzichtet werden, so dass das gesamte OP-Umfeld übersichtlicher gestaltet ist. Das Instrument ist einfach zu bedienen, weil ein Anwender lediglich mit dem Zusammenführen der Griffeinrichtungen bis zum vollständigen Schließen der Branchen fortfahren muss, um nach dem Arretieren des Gewebes die Stromzuführung über die Schalteinrichtung einzuleiten. Damit lässt sich der Beginn eines Koagulationsvorganges und/oder eines Schneidvorganges präzise in Verbindung mit der Positionierung des zu behandelnden Gewebes abstimmen. Eine Deaktivierung des HF-Stromes bzw. eine Unterbrechung der Stromzufuhr kann z B. dadurch erfolgen, dass die Schalteinrichtung wieder "losgelassen" wird. Das heißt, dass die Stromzufuhr nur so lange aufrecht erhalten wird, so lange die Schalteinrichtung "gedrückt", also betätigt ist und eine bestimmte Kraft über die Griffeinrichtungen übermittelt wird. Möglich ist es auch, dass eine Deaktivierung der Stromzufuhr durch erneute Betätigung der Schalteinrichtung oder ggf. durch zusätzliche Schalteinrichtungen erfolgt.

Vorzugsweise kann auch das Maulteil, also können die Elektrodenteile zum Fassen des Gewebes nur so lange geschlossen bleiben, wie im Prinzip Kraft über die Griffeinrichtungen auf die Elektrodenteile ausgeübt wird. Dies ist vor allem bei schnellem Arbeiten von Vorteil. Gegebenenfalls werden die Elektrodenteile dann durch einen Rückstellmechanismus, z. B. durch eine Feder, wieder in ihre geöffnete Position gezwungen, sofern die Griffeinrichtungen nicht betätigt werden. Möglich ist es jedoch auch, dass ein Rastmechanismus die geschlossenen Elektrodenteile fixiert oder dass das Instrument derart ausgebildet ist, dass das Elektrodenteil oder die Elektrodenteile ohne zusätzliche Einrichtung in der gewünschten Position verbleibt bzw. verbleiben.

Die soeben beschriebene Aktivierung des hochfrequenten Stromes lässt sich vorzugsweise bei elektrochirurgischen Instrumenten vorsehen, die z. B. als Schereninstrumente für die offene Chirurgie ausgebildet sind. Auch lassen sich Instrumente für die Endoskopie, z. B. laparoskopische Instrumente, unproblematisch wie oben beschrieben ausbilden, so dass deren einfache und sichere Handhabung gewährleistet ist. Laparoskopische Instrumente weisen - ebenso wie beispielsweise bipolare Scheren - Branchen auf, die z. B. scherenähnlich betätigbar sind und über die die Elektrodenteile bewegt werden können. In der Praxis sind die Branchen im proximalen Bereich meist derart ausgebildet, dass nur eine Branche bewegbar ist, während die andere Branche z. B. integral mit einem Gehäuse und daher fixiert ausgebildet ist. Zur Betätigung der Branchen und damit der Elektrodenteile sind an einem proximalen Bereich der Branchen Griffeinrichtungen vorgesehen, während die Elektrodenteile an einem distalen Bereich zum Fassen von Gewebe und zum Durchleiten von hochfrequentem Strom durch das Gewebe ausgebildet sind. Um nun minimal-invasive Eingriffe durchführen zu können, werden die Branchen über einen Umlenkmechanismus derart verlängert, dass sich die Bewegung der Branchen über die Griffeinrichtungen durch den Umlenkmechanismus auf die Elektrodenteile übertragen lässt. Der Umlenkmechanismus ist z. B. innerhalb eines Rohrschaftes angeordnet, der zwischen dem proximalen Bereich der Branchen und dem distalen Bereich ausgebildet ist. Der Rohrschaft ermöglicht es nun, die Elektrodenteile in eine Körperhöhle einzuführen, z. B. in die Bauchhöhle, wobei die Betätigung der Elektrodenteile von "außen" über die Branchen im proximalen Bereich mittels der Griffeinrichtungen ausgeführt werden kann. Beabstandungselement, elastisch verformbares Element und Schalteinrichtung sind bei derartigen laparoskopischen Instrumenten dann ebenfalls wie bei einem Schereninstrument für die offene Chirurgie angeordnet.

In einer ersten bevorzugten Ausführungsform ist das elastisch verformbare Element zwischen Schalteinrichtung und Beabstandungselement im proximalen Bereich der Branchen angeordnet, so dass sich die Weiterbewegung der entsprechenden Griffeinrichtung zur Betätigung der Schalteinrichtung unproblematisch durchführen lässt. Dabei kann das Beabstandungselement sowohl im proximalen Bereich der Branchen, als auch im distalen Bereich, also nahe den Elektrodenteilen oder sogar an diesen angeordnet sein.

Das Beabstandungselement muss jedoch derart ausgebildet sein, dass der gewünschte Minimalabstand zwischen den Elektrodenteilen definiert wird, wobei bei Erreichen des Minimalabstandes die Weiterbewegung der Griffeinrichtung im proximalen Bereich möglich sein muss, ohne dass die Elektrodenteile in ihrer Position verändert werden.

In allen Ausführungsformen der Erfindung ist das elastisch verformbare Element an der Branche oder an mindestens einer der Griffeinrichtungen, diese durchsetzend angeordnet und derart ausgebildet, dass durch das elastisch verformbare Element eine Soll-Biege- oder -Knickstelle an der Branche oder der Griffeinrichtung vorgesehen ist. Die Soll-Biege- oder -Knickstelle erlaubt eine Biegung bzw. Knickung mindestens der entsprechenden Griffeinrichtung oder auch mindestens einer Branche an gewünschter Stelle, ohne die übrigen Bereiche über Gebühr, z. B. auf Biegung, zu beanspruchen. So lässt sich auf einfache Weise der mindestens eine Bereich der Griffeinrichtung zur Betätigung der SchaIteinrichtung weiterbewegen.

Eine bevorzugte Ausführungsform sieht vor, dass das elastisch verformbaren Element als ein Branchenabschnitt oder ein Griffeinrichtungsabschnitt vorgesehen ist, der gegenüber umgebenden Branchenbereichen oder Griffeinrichtungsbereichen verjüngt ausgebildet ist. Auf diese Weise lässt sich ohne großen Aufwand die Soll-Biege- oder -Knickstelle an mindestens einer Branche oder an mindestens einer der Griffeinrichtungen ausführen und die gewünschte Biegung oder Knickung ist unproblematisch durchführbar. Da Branche und elastisch verformbares Element integral miteinander ausgebildet sind, ist das Instrument einfach herstellbar und lässt sich auch nach einem Eingriff problemlos reinigen.

Grundsätzlich können beide Branchen oder Griffeinrichtungen mit einem elastisch verformbaren Element ausgebildet sein oder nur eine der Branchen oder Griffeinrichtungen, nämlich die die Schalteinrichtung aufweisende Branche oder Griffeinrichtung oder die gegenüberliegende Branche oder Griffeinrichtung. In jedem Falle werden die Griffeinrichtungen und ggf. auch die Branchen aufgrund des elastisch verformbaren Elements zur Betätigung der Schalteinrichtung aneinander geführt. Dabei können neben Biegeelementen auch dehn- oder streckbare Elemente, stauchbare Elemente oder dergleichen Einrichtungen vorgesehen sein. Das elastisch verformbare Element ist immer derart in den Kraftübertragungsweg zu integrieren, dass beide Wegbereiche von dem mindestens einen Bereich der Griffeinrichtungen durchlaufen werden können.

Eine erfindungsgemäße Lösung sieht vor, dass das Beabstandungselement als Anschlagelement an mindestens einer Branche ausgebildet ist. Das Anschlagelement ist als Vorsprung an einer der Branchen oder an beiden Branchen angeordnet, so dass ein Zusammenführen der Branchen unter den Minimalabstand durch das Anschlagelement verhindert wird. Damit ist auf einfachste Weise ein Kurzschluss zwischen den Elektrodenteilen zu vermeiden, gleichzeitig wird durch das Beabstandungselement der erste "Wegbereich" definiert, so dass die Weiterbewegung der entsprechenden Griffeinrichtung auch bei Erreichen des Minimalabstandes zwischen den Elektrodenteilen über den zweiten "Wegbereich" gewährleistet ist.

Eine vorteilhafte Ausführungsform sieht vor, dass die Schalteinrichtung und die gegenüberliegende Branche oder Griffeinrichtung derart ausgebildet sind, dass die Schalteinrichtung durch Aufsetzen der gegenüberliegenden Branche bzw. Griffeinrichtung betätigbar ist. Das heißt also, dass die Schalteinrichtung lediglich durch Kontaktierung mit der gegenüberliegenden Branche bzw. Griffeinrichtung betätigt wird. Dies ist eine besonders einfache und kostengünstige Anordnung zur Aktivierung des hochfrequenten Stromes.

Vorteilhafterweise ist ein an der der Schalteinrichtung gegenüberliegenden Branche oder Griffeinrichtung ausgebildetes Betätigungselement, z. B. ein Betätigungszapfen, vorgesehen, der derart angeordnet ist, dass er die Schalteinrichtung bei Weiterbewegung des mindestens einen Bereichs der Griffeinrichtungen, z. B. bei Biegung oder Knickung mindestens einer Branche oder Griffeinrichtung, betätigt. Dies ist insbesondere dann von Vorteil, wenn die Schalteinrichtung z. B. innerhalb der gegenüberliegenden Branche oder Griffeinrichtung zum Schutz vor einer versehentlichen Betätigung angeordnet ist. Die Schalteinrichtung ist dann ausschließlich durch den Zapfen erreichbar und somit betätigbar.

Vorzugsweise ist ein die Schalteinrichtung umgebendes Abdeckungselement mit einem in Richtung des Betätigungselements ausgebildeten Öffnungsbereich vorgesehen. Das Betätigungselement bzw. der Betätigungszapfen betätigt die Schalteinrichtung dann durch den Öffnungsbereich hindurch. Damit lässt sich, wie auch in der zuvor beschriebenen Ausführungsform, eine versehentliche Betätigung der Schalteinrichtung bei nicht zusammengeführten Branchen vermeiden, weil letztendlich nur das speziell ausgebildete Betätigungselement Zugang zur Schalteinrichtung hat. Dies erhöht die Sicherheit für den Patienten während des Eingriffs, weil keine ungewollte Stromzufuhr in das zu behandelnde Gewebe erfolgen kann.

Die Schalteinrichtung ist in einer vorteilhaften Ausführungsform als Schalter, Taster oder dergleichen Element ausgebildet. Somit lässt sich die Anordnung auf einfache Weise mit herkömmlichen Bauelementen realisieren.

In einer bevorzugten Ausführungsform ist die Schalteinrichtung als Reedkontakt ausgebildet, der z. B. innerhalb der entsprechenden Branche oder Griffeinrichtung angeordnet ist. Zur Betätigung des Reedkontaktes ist ein Magnetelement an der Branche oder der Griffeinrichtung vorgesehen, die dem Reedkontakt gegenüberliegt. Durch das Biegen bzw. Knicken der entsprechenden Branche bzw. Weiterbewegen des mindestens einen Bereichs der Griffeinrichtung nach Erreichen des Minimalabstandes lassen sich dann berührungslos der Schalter, also der Reedkontakt, und damit die Stromzufuhr aktivieren. Dies stellt eine besonders bedienerfreundliche Ausführungsform dar, zumal auch eine Reinigung des Instruments aufgrund des im Inneren des Instruments angeordneten Schalters problemlos durchgeführt werden kann. Außerdem werden durch den im Inneren des Instruments angeordneten Schalter keine Kriechwege für Flüssigkeiten und sonstige Verschmutzungen am und in das Instrument bereitgestellt.

Die oben genannte Aufgabe wird ferner durch ein elektrochirurgisches Instrument nach Patentanspruch 9 gelöst.

Insbesondere wird die Aufgabe durch ein elektrochirurgisches Instrument zum Koagulieren und/oder Schneiden biologischen Gewebes gelöst, das Folgendes umfasst:
- einen Schaft,
- mindestens eine erste Griffeinrichtung und eine zweite Griffeinrichtung an einem proximalen Bereich des Instruments zu dessen Handhabung, wobei die Griffeinrichtungen gegeneinander bewegbar sind,
- mindestens ein Elektrodenteil an einem distalen Bereich des Instruments zum Durchleiten eines HF-Stromes durch das Gewebe, wobei das Elektrodenteil mittels der Griffeinrichtungen von einer Ruheposition in eine Arbeitsposition bringbar ist,
- Stromzuführungseinrichtungen zum Zuführen des HF-Stromes zu dem mindestens einen Elektrodenteil von einem HF-Generator,
- eine Schalteinrichtung zum Aktivieren des HF-Stromes bei zusammengeführten Griffeinrichtungen,
- mindestens ein elastisch verformbares Element, das derart an und/oder in dem Instrument angeordnet ist, dass mindestens ein Bereich der Griffeinrichtungen zur Betätigung der Schalteinrichtung weiter bewegbar ist, wenn sich das Elektrodenteil in der Arbeitsposition befindet.

Ein weiterer wesentlicher Punkt der Erfindung liegt folglich auch darin, dass das elastisch verformbares Element und die Schalteinrichtung derart ausgebildet und in Bezug zueinander angeordnet sind, dass die Schalteinrichtung bei einem Weiterbewegen des mindestens einen Bereichs der Griffeinrichtungen betätigbar ist. Dabei durchläuft der Bereich der Griffeinrichtungen ebenfalls verschiedene "Wegbereiche", wie es oben bereits in ähnlicher Weise erläutert wurde. Das Durchlaufen eines ersten "Wegbereichs" (beim Gegeneinanderbewegen der Griffeinrichtungen) dient der Bewegung des mindestens einen Elektrodenteils, um dieses in die Arbeitsposition zu bringen. Sobald die Arbeitsposition erreicht ist, kann der entsprechende Bereich der Griffeinrichtungen mindestens aufgrund des elastisch verformbaren Elements derart weiter bewegt werden, dass die Betätigung der Schalteinrichtung ermöglicht wird. Die entsprechende Griffeinrichtung durchläuft dabei einen zweiten "Wegbereich", um das Betätigen der Schalteinrichtung zur Aktivierung des HF-Stromes - über die Griffeinrichtung selbst oder ein zusätzliches Bauteil - zu ermöglichen. Die Stromzuführungseinrichtungen sind bei Instrumenten dieser Art im Prinzip ähnlich ausgebildet, wie es bei den oben beschriebenen Brancheninstrumenten vorgesehen ist. Auch hier bildet die Schalteinrichtung einen Teil der Stromzuführungseinrichtungen und kann erfindungsgemäß auf einfachste Art betätigt werden.

Auch mit dieser Anordnung kann auf aufwändige Hand- oder Fußschalter verzichtet werden, so dass das gesamte OP-Umfeld übersichtlicher gestaltet ist. Zudem lässt sich der Beginn eines Koagulationsvorganges und/oder eines Schneidvorganges präzise in Verbindung mit der Positionierung der Elektrode an dem zu behandelnden Gewebe abstimmen.

Auch bei Instrumenten dieser Bauart kann eine Deaktivierung der Stromzufuhr wie oben beschrieben erfolgen. Das Verbleiben des Elektrodenteils in der Arbeitsposition bzw. des Maulteils in der geschlossenen Position kann ebenfalls derart gewährleistet werden, wie es bereits bei oben beschriebenen Instrumenten vorgesehen ist. So kann z. B. ein Rastmechanismus zur Beibehaltung der Arbeitsposition dienen oder aber es ist vom Anwender aktiv Kraft aufzuwenden, um die Arbeitsposition beizubehalten.

Die soeben beschriebene Aktivierung des hochfrequenten Stromes lässt sich sowohl bei Instrumenten für die offene Chirurgie als auch bei Instrumenten für die Endoskopie, z. B. bei laparoskopischen Instrumenten vorsehen. Auch lassen sich Instrumente für die Endoskopie, z. B. laparoskopische Instrumente, unproblematisch wie oben beschrieben ausbilden, so dass deren einfache und sichere Handhabung gewährleistet ist. Laparoskopische Instrumente weisen - ebenso wie beispielsweise bipolare Scheren - Branchen auf, die z. B. scherenähnlich betätigbar sind und über die die Elektrodenteile bewegt werden können.

In einer bevorzugten Ausführungsform ist innerhalb des Schaftes ein über die Griffeinrichtungen bewegbares Trägerelement zum Tragen des mindestens einen Elektrodenteils und zum Bewegen des Elektrodenteils von der Ruheposition in die Arbeitsposition angeordnet. Das Trägerelement ist z. B. als eine Art Schubstange ausgebildet und ermöglicht die Positionierung des Elektrodenteils über die Griffeinrichtungen an dem zu behandelnden Gewebe. Die Ruheposition des Elektrodenteils ist dabei vorzugsweise als diejenige Position zu verstehen, bei der das Elektrodenteil innerhalb des Schaftes platziert und von außen im Wesentlichen unzugänglich ist. Eine Bewegung des Trägerelements in Erstreckungsrichtung des Schaftes ermöglicht das "Ausfahren" des Elektrodenteils aus dem Schaft, so dass eine Positionierung an dem zu behandelnden Gewebe ermöglicht wird.

Weist ein Instrument zwei Elektroden auf, die z. B. als Maulteil eine bipolare Anordnung ausbilden, so lassen sich diese Elektroden ebenfalls von einer Ruhe- in eine Arbeitsposition mittels des Trägerelements überführen. Hierbei kann die Ruheposition z. B. das geöffnete Maulteil bedeuten, während die Arbeitsposition über das geschlossene Maulteil definiert ist. Um ein Schließen des Maulteils zu ermöglichen, wird das Maulteil in Erstreckungsrichtung des Schaftes, hin zum proximalen Ende des Instruments bewegt, wobei das Einfahren der Elektrodenteile in den Schaft das Schließen des Maulteils bewirkt.

Vorzugsweise ist ein bei einem Zusammenführen der Griffeinrichtungen wirksam werdendes Beabstandungselement derart in oder an dem Instrument vorgesehen, dass durch dieses die Arbeitsposition des mindestens einen Elektrodenteils festlegbar ist. Es wird also auch hier über das Beabstandungselement der erste Wegbereich einer Griffeinrichtung definiert. Das Trägerelement weist dazu beispielsweise mindestens eine erste Anschlageinrichtung als Beabstandungselement auf, die beispielsweise aus dem Trägerelement hervorsteht und mit mindestens einem ersten Schaftvorsprung derart zusammenwirkt, dass durch das Zusammenwirken die Arbeitsposition des Elektrodenteils festlegbar ist. Die Anschlageinrichtung ist derart an dem Trägerelement angeordnet; dass sie mit dem Trägerelement über die Griffeinrichtungen bewegbar ist. Der Schaftvorsprung kann z. B. innerhalb des Schaftes angeordnet sein und aus diesem in das Innere des Schaftes hinein hervorstehen. So wirkt der Schaftvorsprung als Stoppelement, sobald die Anschlageinrichtung den Schaftvorsprung erreicht hat.

In einer bevorzugten Ausführungsform ist das elastisch verformbare Element derart an dem Trägerelement angeordnet und das Trägerelement weist mindestens eine zweite Anschlageinrichtung auf, die mit mindestens einem zweiten, die Schalteinrichtung aufweisenden Schaftvorsprung derart zusammenwirkt, dass die Schalteinrichtung bei Weiterbewegung des mindestens einen Bereichs der Griffeinrichtungen in Erstreckungsrichtung des Schaftes mindestens durch Verformung des elastisch verformbaren Elements mittels der zweiten Anschlageinrichtung betätigbar ist. Vorzugsweise ist das elastisch verformbare Element derart ausgebildet, dass es stauchbar ist. Bei diesem Ausführungsbeispiel kann das Trägerelement aus zwei Abschnitten bestehen, die über das elastisch verformbare Element miteinander in Verbindung stehen. Ein distaler Abschnitt weist die erste Anschlageinrichtung auf, die mit dem ersten Schaftvorsprung beim "Ausfahren" des Elektrodenteils zusammenwirkt, so dass die Arbeitsposition des Elektrodenteils festgelegt ist. Ein proximaler Abschnitt weist die zweite Anschlageinrichtung auf, wobei das Weiterbewegen des entsprechenden Griffteils ein Weiterbewegen des proximalen Abschnitts bewirkt, indem sich zumindest das elastisch verformbare Element verformt. Vorzugsweise wird also der proximale Abschnitt in Richtung des distalen Abschnitts bewegt, wobei die zweite Anschlageinrichtung die an dem zweiten Schaftvorsprung angeordnete Schalteinrichtung betätigt. Eine weitere Bewegung des distalen Abschnitts wird hier durch die erste Anschlageinrichtung verhindert, d. h., der proximale Abschnitt ist auch dann bewegbar, wenn sich der distale Abschnitt im Stillstand befindet. Das elastisch verformbare Element ist jedoch derart auszulegen, dass die beiden Abschnitte des Trägerelements trotz Kopplung über das elastisch verformbare Element im Gleichlauf - während des Durchlaufens des ersten Wegbereichs der entsprechenden Griffeinrichtung - verschiebbar sind und so ein Übergang von der Ruheposition des Elektrodenteils in die Arbeitsposition möglich ist. Der zweite Wegbereich, über den der mindestens eine Bereich der Griffeinrichtungen verfahren wird, kann erst dann durchlaufen werden, wenn auf das elastisch verformbare Element eine hinreichend große Kraft ausgeübt wird, um eine Verformung zu ermöglichen. Dies wird u. a. dadurch gewährleistet, dass sich der distale Abschnitt des Trägerelements bereits im Anschlag befindet. Das heißt, die Betätigung der Schalteinrichtung über den proximalen Abschnitt erfolgt, obwohl der distale Abschnitt nicht mehr bewegt wird; der proximale Abschnitt schiebt sich gegen den distalen Abschnitt. Um ein "Einfahren" des Elektrodenteils in den Schaft zu gewährleisten, d. h., das Elektrodenteil von der Arbeitsposition wieder in die Ruheposition zu überführen, ist beispielsweise ein Rückhaltemechanismus vorgesehen, der die beiden Abschnitte während des Einfahrens zusammenhält. Somit wird ggf. eine Überdehnung des elastisch verformbaren Elements vermieden.

Die hier beschriebene erste Anschlageinrichtung, die mit dem ersten Schaftvorsprung zusammenwirkt, entspricht im Prinzip dem oben erläuterten Beabstandungselement. Eine Art Beabstandungselement, Anschlageinrichtung oder dergleichen Einrichtung ist auch bei Rohrschaftinstrumenten der hier beschriebenen Art vorteilhaft, um die beiden Wegbereiche festzulegen und so eine Weiterbewegung des mindestens einen Bereichs der Griffeinrichtungen auch ohne Weiterbewegung des mindestens einen Elektrodenteils zu ermöglichen.

Vorteilhafterweise sind das Trägerelement und das elastisch verformbare Element mittels eines entlang dem Schaft verlaufenden, mit dem Trägerelement verbundenen Schubelements bewegbar, wobei die erste Griffeinrichtung zum Halten des Instruments und die zweite Griffeinrichtung zum Betätigen des Schubelements vorgesehen ist. Das Schubelement überträgt also die Bewegung der entsprechenden Griffeinrichtung auf das Trägerelement, um das Elektrodenteil von der Ruheposition in die Arbeitsposition zu befördern und umgekehrt und zudem die Betätigung der Schalteinrichtung zu ermöglichen. Ist das Schubelement außen am Schaft entlangführbar, ist vorzugsweise ein Mechanismus vorzusehen, der die Bewegung des Trägerelements für den Anwender auf einfache Weise ermöglicht. Das Schubelement weist dazu vorzugsweise ein mit diesem verbundenes Daumenelement als die zweite Griffeinrichtungen auf, so dass der Anwender die Griffeinrichtung über beide Wegbereiche durch ledigliche Daumenbewegung verfahren kann. Über die erste Griffeinrichtung, z. B. einen am Schaft angeordneten Handgriff, hält der Anwender das Instrument. Möglich ist es auch, das Schubelement mittels eines Branchenelements als die zweite Griffeinrichtung zu bewegen, wobei das Branchenelement an einem ersten Ende an dem Handgriff des Schaftes angelenkt und an einem zweiten Ende mit dem Schubelement über ein an diesem und an dem Branchenelement (52b) angelenktes Hebelelement (52c) verbunden ist. Die Bewegung des Schubelements erfolgt dann für den Anwender derart, als ob dieser eine Schere bewegen würde. Beide Bewegungsmechanismen ermöglichen eine präzise Betätigung des Trägerelements als auch der Schalteinrichtung.

Vorzugsweise ist das elastisch verformbare Element als ein Schraubenfederelement ausgebildet. Bei entsprechender Auslegung des Schraubenfederelements ist gewährleistet, dass die beiden Abschnitte des Trägerelements unproblematisch im Gleichlauf bewegt werden können, wenn das Elektrodenteil von der Ruhe- in die Arbeitsposition geführt werden soll. Zudem erlaubt die Schraubenfeder auf einfache Weise die Weiterbewegung der Griffeinrichtung zur Betätigung der Schalteinrichtung. Im Übrigen sind auch andere Federarten verwendbar, so z. B. Blattfedern.

Auch bei Schaftinstrumenten dieser Art wird eine Weiterbewegung der entsprechenden Griffeinrichtung über ein elastisch verformbares Element erfolgen, das eine Soll-Biege- oder -Knickstelle ausbildet. So ist das elastisch verformbare Element an mindestens einer Griffeinrichtung, diese durchsetzend angeordnet und derart ausgebildet , dass durch das elastisch verformbare Element eine Soll-Biege- oder -Knickstelle an der Griffeinrichtung vorgesehen ist. Vorzugsweise kann das elastisch verformbare Element auch als ein Griffeinrichtungsabschnitt vorgesehen sein, der gegenüber umgebenden Griffeinrichtungsbereichen verjüngt ausgebildet ist, so dass durch den Griffeinrichtungsabschnitt eine Soll-Biege- oder -Knickstelle an der Griffeinrichtung vorgesehen ist. Die Soll-Biege- oder -Knickstelle erlaubt auf einfache Weise eine Biegung bzw. Knickung der Griffeinrichtung an gewünschter Stelle, ohne die übrigen Bereiche über Gebühr, z. B. auf Biegung, zu beanspruchen. Ein verjüngter Knick- bzw. Biegebereich wird in der Regel integral mit der entsprechenden Griffeinrichtung ausgebildet sein, so dass das Instrument einfach herstellbar ist und sich auch nach einem Eingriff problemlos reinigen lässt. Das Beabstandungselement ist dann derart angeordnet, dass durch dieses der erste Wegbereich der entsprechenden Griffeinrichtung definiert wird, d. h., bei Zusammenführen der Griffeinrichtungen und Erreichen des Beabstandungselements ist auch die Arbeitsposition des Elektrodenteils erreicht. Danach lässt sich eine der Griffeinrichtungen nur zur Betätigung der Schalteinrichtung weiterbewegen.

Unterschiedliche Materialien von Griffeinrichtung (oder auch Branche, vgl. oben beschriebene Brancheninstrumente) und elastisch verformbarem Element und/oder unterschiedliche Querschnitte erlauben das Festlegen einer definierten Weiterbewegung des mindestens einen Bereichs der Griffeinrichtungen. Die Ausbildung des elastisch verformbaren Elements als lediglich verjüngter Bereich, der jedoch aus dem Branchen - oder Griffeinrichtungsmaterial besteht, ermöglicht zusätzlich eine einfache und leicht zu handhabende Ausführungsform des erfindungsgemäßen Gegenstandes.

Auch lässt sich das elastisch verformbare Element als ein Stauchelement an der entsprechenden Griffeinrichtung ausbilden, so dass die Betätigung der Schalteinrichtung durch ein Stauchen der Griffeinrichtung ermöglicht wird.

Erfindungsgemäß kann die Schalteinrichtung als Schalter, Taster oder dergleichen Element ausgebildet sein, wie bereits oben näher erläutert. Handelsübliche Bauteile dieser Art ermöglichen eine einfache Herstellung des hier in Rede stehenden Instruments.

Die Schalteinrichtung lässt sich vorzugsweise auch als Reedkontakt ausbilden, wobei dann ein Magnetelement zur Betätigung des Reedkontaktes an mindestens einer Griffeinrichtung oder an der zweiten Anschlageinrichtung angeordnet ist. Durch das Weiterbewegen der Griffeinrichtung und ggf. auch der Anschlageinrichtung lässt sich der Reedkontakt berührungslos schalten. Die Vorteile dieser Ausführungsform sind - wie bereits oben näher erläutert - insbesondere bei der Herstellung des Instruments zu sehen. So kann bei berührungsloser Schaltmöglichkeit eine vollständige Abdeckung der Schalteinrichtung vorgesehen werden. Damit lassen sich Kriechwege für Flüssigkeiten und sonstige Verschmutzungen vermeiden.

Wie bereits erwähnt, lässt sich das erfindungsgemäße Instrument sowohl für die offene Chirurgie, als auch für die Endoskopie und ebenso für die monopolare, als auch bipolare Technik ausbilden.

Vorzugsweise ist das elastisch verformbare Element aus einem Material ausgebildet, das neben seiner Elastizität eine hohe Verschleißfestigkeit aufweist. Hier kommt beispielsweise ein Polyetheretherketon (PEEK) oder dergleichen Kunststoff in Betracht. Polyetherketone (PEK) sind hochtemperaturbeständig und gegen die meisten organischen und anorganischen Chemikalien beständig. Auch kann das elastisch verformbare Element aus einem Federstahl ausgebildet sein. Handelsübliche Federelemente dieser Art ermöglichen eine einfache und kostengünstige Herstellung des Instruments.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- Fig. 1 eine bevorzugte Ausführungsform eines erfindungsgemäßen elektrochirurgischen Instruments in einer Seitenansicht;
- Fig. 2 eine weitere bevorzugte Ausführungsform des erfindungsgemäßen elektrochirurgischen Instruments in einer Seitenansicht;
- Fig. 3 eine vereinfachte Darstellung einer weiteren bevorzugten Ausführungsform des erfindungsgemäßen elektrochirurgischen Instruments in einer Seitenansicht;
- Fig. 4 eine vereinfachte Darstellung einer weiteren bevorzugten Ausführungsform des erfindungsgemäßen elektrochirurgischen Instruments in einer Seitenansicht;
- Fig. 5 einen Ausschnitt aus einer weiteren bevorzugten Ausführungsform des erfindungsgemäßen elektrochirurgischen Instruments, nämlich eine Schalteinrichtung mit Betätigungsmöglichkeit;
- Fig. 6 einen Ausschnitt aus einer weiteren bevorzugten Ausführungsform des erfindungsgemäßen elektrochirurgischen Instruments, nämlich eine weitere Schalteinrichtung mit Betätigungsmöglichkeit;
- Fig. 7 eine weitere bevorzugte Ausführungsform des erfindungsgemäßen elektrochirurgischen Instruments in einer Seitenansicht;.
- Fig. 8 eine vereinfachte Darstellung einer weiteren bevorzugten Ausführungsform des erfindungsgemäßen elektrochirurgischen Instruments in einer Seitenansicht;
- Fig. 9 eine weitere bevorzugten Ausführungsform des erfindungsgemäßen elektrochirurgischen Instruments in einer perspektivischen Ansicht;
- Fig. 10 einen Ausschnitt aus einem erfindungsgemäßen elektrochirurgischen Instrument, nämlich einen Ausschnitt aus einem Trägerelement mit einem elastisch verformbaren Element in Seitenansicht, wie es in den Instrumenten gemäß der Fig. 9, 11, 12 und 13 angewendet werden kann;
- Fig. 11 einen Ausschnitt aus einer weiteren bevorzugten Ausführungsform des erfindungsgemäßen elektrochirurgischen Instruments, nämlich einen proximalen Bereich des Instruments in perspektivischer Ansicht;
- Fig. 12 einen Ausschnitt aus einer weiteren bevorzugten Ausführungsform des erfindungsgemäßen elektrochirurgischen Instruments, nämlich einen proximalen Bereich des Instruments in perspektivischer Ansicht;
- Fig. 13 eine weitere bevorzugte Ausführungsform des erfindungsgemäßen elektrochirurgischen Instruments in einer Seitenansicht;
- Fig. 14 einen Ausschnitt aus einer weiteren bevorzugten Ausführungsform des erfindungsgemäßen elektrochirurgischen Instrument in vereinfachter Darstellung, nämlich einen proximalen Bereich des Instruments mit Schalteinrichtung in Seitenansicht;
- Fig. 15 ein distales Ende eines Rohrschaftes gemäß einer bevorzugten Ausführungsform mit einem geöffneten Maulteil;
- Fig. 16 das distale Ende des Rohrschaftes gemäß Fig. 15 mit geschlossenem Maulteil.

In der nachfolgenden Beschreibung werden für gleiche und gleich wirkende Teile dieselben Bezugsziffern verwendet.

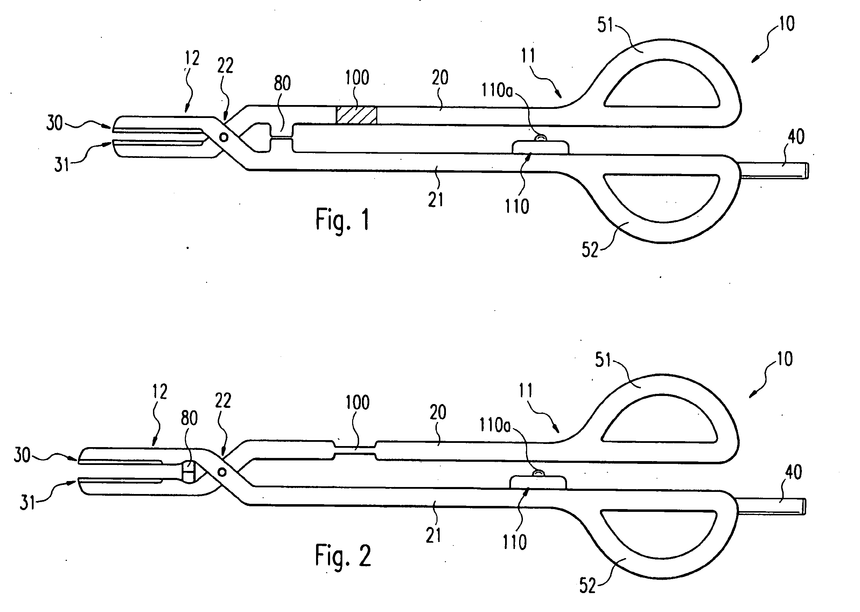

Fig. 1 zeigt eine bevorzugte Ausführungsform eines erfindungsgemäßen elektrochirurgischen Instruments 10 für die offene Chirurgie mit einer Anordnung zum Aktivieren von hochfrequentem Strom. Das Instrument ist in Seitenansicht dargestellt. In der Abbildung sind mit den Bezugsziffern 20 und 21 zwei Branchen des elektrochirurgischen Instruments 10 bezeichnet. Die beiden Branchen 20, 21 sind über eine Gelenkverbindung 22 miteinander verbunden und durch diese gegeneinander bewegbar, hier z. B. verschwenkbar. Die Gelenkverbindung 22 teilt das Instrument 10 bzw. die Branchen 20, 21 im Prinzip in einen proximalen Bereich 11 und einen distalen Bereich 12 auf. Im proximalen Bereich 11 sind an die Branchen 20, 21 anschließende Griffeinrichtungen 51, 52 zur Handhabung des Instruments 10 vorgesehen. Im distalen Bereich 12 sind an den Branchen 20, 21 einander gegenüberliegende Elektrodenteile 30, 31 angeordnet, mittels derer sich beispielsweise ein Gefäß oder Gewebe fassen und durch Zuleitung von hochfrequentem Strom koagulieren, ggf. auch schneiden lässt. Die Zuleitung des Stromes erfolgt über ein an einer der Branchen 21 ausgebildetes Stromanschlusselement bzw. über eine Stromzuführungseinrichtüng 40 zum Anschließen des elektrochirurgischen Instruments 10 an einen (nicht dargestellten) HF-Generator. Der HF-Strom wird dann beispielsweise durch in dem Instrument 10 laufende elektrische Leitungen (nicht gezeigt) den Elektrodenteilen 30, 31 zugeführt.

Im proximalen Bereich 11 des Instruments 10 ist ein Beabstandungselement 80 angeordnet, das aus zwei sich gegenüberliegenden Anschlagelementen ausgebildet ist. Die Anschlagelemente sind integral mit den Branchen 20, 21 vorzugsweise auch aus demselben Material ausgebildet. Durch das Beabstandungselement 80 ist ein Minimalabstand zwischen den Elektrodenteilen 30, 31 nicht unterschreitbar, so dass ein Kurzschluss zwischen den Elektrodenteilen 30, 31 bei zusammengeführten Branchen 20, 21 vermieden wird. Das Beabstandungselement 80 verhindert zudem ein vollständiges Zusammenführen der Branchen 20, 21 auch im proximalen Bereich 11.

Eine der Branchen 21 weist eine Schalteinrichtung 110, hier z. B. einen Taster 110a, als Teil der Stromzuführungseinrichtungen auf, der derart angeordnet ist, dass er durch Kontaktierung mit der gegenüberliegenden Branche 20 betätigt werden könnte. Die Betätigung aktiviert die Stromzufuhr (von dem HF-Generator), so dass die Elektrodenteile zu Behandlung von Gewebe eingesetzt werden können. Da das Beabstandungselement 80 nun ein weiteres Zusammenführen der Branchen 20, 21 bei Erreichen des Minimalabstandes zwischen den Elektrodenteilen 30, 31 auch im proximalen Bereich 11 verhindert, ist an der der Schalteinrichtung 110 gegenüberliegenden Branche 20 ein elastisch verformbares Element 100 vorgesehen. Das elastisch verformbare Element 100 ermöglicht nun ein Biegen oder auch Knicken der Branche 20 zur Betätigung des Tasters 110a auch bei zusammengeführten Branchen 20, 21 und zusammengeführten Anschlagelementen des Beabstandungselements 80. Die Branchen 20, 21 bzw. die Griffeinrichtungen 51, 52 lassen sich also auch bei Erreichen des Minimalabstandes weiter zusammenführen, d. h., die das elastisch verformbare Element 100 aufweisende Branche 20 bzw. die Griffeinrichtung 51 ist in Richtung der Schalteinrichtung 110 weiter bewegbar, hier z. B. biegbar.

Die Griffeinrichtung 51 durchläuft demnach - zusammen mit den Branchen - beim Zusammenführen einen ersten "Wegbereich", bis zum Erreichen des Minimalabstandes, der durch das Beabstandungselement 80 definiert ist. Da das Beabstandungselement 80 ein vollständiges Zusammenführen der Branchen 20, 21 auch im proximalen Bereich 11 verhindert, kann ein zweiter "Wegbereich" der Griffeinrichtung 51 nur aufgrund des elastisch verformbaren Elements 100 durchlaufen werden, wobei so die Betätigung der Schalteinrichtung 110 ermöglicht wird. Beabstandungselement 80, elastisch verformbares Element 100 und Schalteinrichtung 110 sind also derart auszubilden und in Bezug zueinander anzuordnen, dass die der Schalteinrichtung 110 gegenüberliegende Branche in Kontakt mit der Schalteinrichtung 110 gebracht werden kann. So lassen sich die Schalteinrichtung 110 betätigen und der hochfrequente Strom zur Behandlung des Gewebes aktivieren.

Das elastisch verformbare Element 100 ist folglich zwischen Beabstandungselement 80 und Schalteinrichtung 110 angeordnet, wobei das Beabstandungselement 80 im proximalen Bereich 11 des Instruments 10 vorgesehen ist. Das elastisch verformbare Element 100 ist beispielsweise aus einem Polyetheretherketon ausgebildet und durchsetzt die Branche 20. Damit ist durch das elastisch verformbare Element 100 eine Soll-Biege - oder -Knickstelle ausbildbar, so dass eine definierte Biegung bzw. Knickung festgelegt werden kann. Die übrigen Branchenbereiche werden somit im Wesentlichen keiner Biegebeanspruchung ausgesetzt.

Fig. 2 zeigt ein elektrochirurgisches Instrument 10 gemäß einer weiteren bevorzugten Ausführungsform. Der Aufbau des Instruments 10 entspricht im Wesentlichen dem in Fig. 1 gezeigten. Das Beabstandungselement 80 ist hier jedoch im distalen Bereich 12 der Branchen 20, 21 angeordnet. Auch das elastisch verformbare Element 100 unterscheidet sich von dem in Fig. 1 gezeigten. Das elastisch verformbare Element 100 ist in diesem Ausführungsbeispiel als verjüngt ausgebildeter Abschnitt gegenüber der übrigen Branche 20 vorgesehen. Dies hat den Vorteil, dass das elastisch verformbare Element 100 integral mit der Branche 20, vorzugsweise auch aus demselben Material ausgebildet werden kann. Damit lässt sich das Instrument 10 auf einfache Weise herstellen. Zudem ist das

Instrument 10 nach einem Eingriff problemlos reinigbar, weil keine Fügekanten oder dergleichen Unterbrechungen an der Branche 20 vorgesehen sind und auch die Verschleißfestigkeit verschiedener Materialien nicht beachtet werden muss.

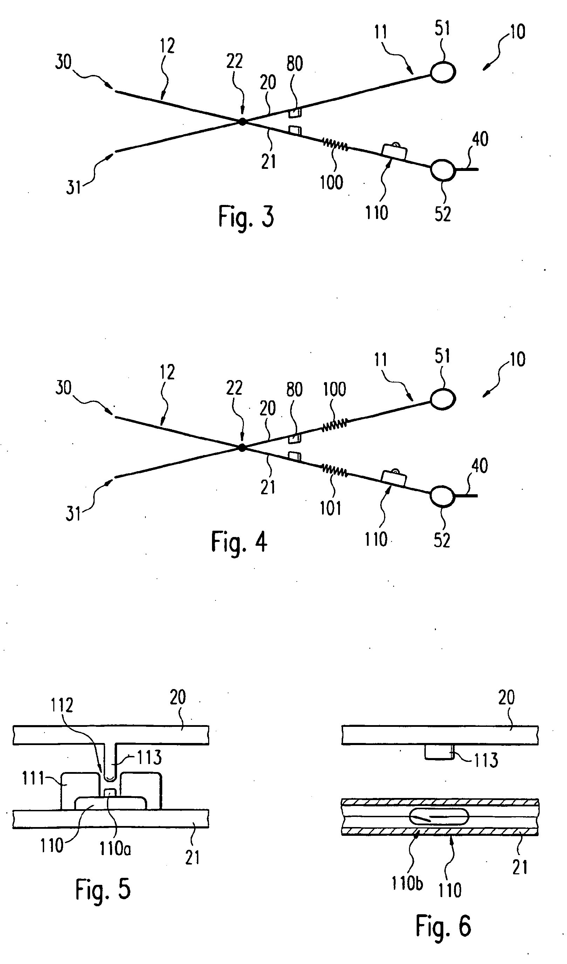

In Fig. 3 ist eine weitere bevorzugte Ausführungsform des erfindungsgemäßen Instruments dargestellt. Die vereinfachte Darstellung zeigt ein Instrument, das im Wesentlichen dem in Fig. 1 beschriebenen entspricht. Lediglich das elastisch verformbare Element 100 ist an der Branche 21 angeordnet, an der auch die Schalteinrichtung 110 vorgesehen ist. Damit ist die Schalteinrichtung 110 zu ihrer Betätigung an die gegenüberliegende Branche 20 heranführbar, indem die entsprechende Griffeinrichtung 52 weiter bewegt wird.

Das in Fig. 4 dargestellte Instrument gemäß einer weiteren bevorzugten Ausführungsform unterscheidet sich zu dem in Fig. 1 gezeigten nur durch ein weiteres elastisch verformbares Element 101, so dass jeweils ein elastisch verformbares Element an jeweils einer der Branchen 20, 21 angeordnet ist. Beide Branchen 20, 21 bzw. die Griffeinrichtungen 51, 52 können dann zur Betätigung der Schalteinrichtung 110 aufeinander zubewegt, also gebogen bzw. geknickt werden.

Fig. 5 und Fig. 6 zeigen jeweils Ausschnitte eines erfindungsgemäßen elektrochirurgischen Instruments 10, wobei Bereiche der Branchen mit entsprechenden Schalteinrichtungen 110 dargestellt sind. Gemäß Fig. 5 ist die Schalteinrichtung 110 als Taster 110a ausgebildet ist. An der gegenüberliegenden Branche 20 ist ein Betätigungselement 113 in Form eines Zapfens angeordnet, das hier beispielsweise integral mit der Branche 20 ausgebildet ist. Das Betätigungselement 113 ermöglicht die gezielte Betätigung des Tasters 110a. Im Gegensatz dazu erfolgt die Betätigung der Schalteinrichtung 110 bei den Ausführungsformen gemäß der Fig. 1 bis 4 über die jeweils gegenüberliegende Branche selbst, indem Schalteinrichtung 110 und gegenüberliegende Branche durch Weiterbewegen der entsprechenden Griffeinrichtung bzw. Griffeinrichtungen miteinander in Kontakt gebracht werden. Wie Fig. 5 zu entnehmen ist, weist die Schalteinrichtung 110 ein sie umgebendes Abdeckungselement 111 mit einem in Richtung des Betätigungselements 113 ausgebildeten Öffnungsbereich 112 auf. Das Betätigungselement 113 betätigt die Schalteinrichtung 110 dann durch den Öffnungsbereich 112 hindurch. Das Abdeckungselement 111 - eine so genannte Kulisse - verhindert eine versehentliche Betätigung der Schalteinrichtung 110, weil der Taster 110a nur für das Betätigungselement 113 zugänglich ist. Dies erhöht die Sicherheit für den Patienten während des Eingriffs, weil keine ungewollte Stromzufuhr in das zu behandelnde Gewebe erfolgen kann.

Fig. 6 zeigt einen in einer der Branchen 21 angeordneten Reedkontakt 110b als Schalteinrichtung 110, der mit einem an der gegenüberliegenden Branche 20 angeordneten Magnetelement als Betätigungselement 113 betätigbar ist. Durch das Weiterbewegen der entsprechenden Griffeinrichtung nach Erreichen des Minimalabstandes lassen sich dann berührungslos der Schalter, also der Reedkontakt 110b, und damit die Stromzufuhr aktivieren. Der innerhalb der Branche 21 vorgesehene Reedkontakt 110b ermöglicht eine problemlose Reinigung des Instruments 10 nach einem Eingriff, weil keine Kriechwege für Flüssigkeiten und sonstige Verschmutzungen am und in das Instrument 10, insbesondere in Umgebung des Schalters bereitgestellt werden. Auch sind keine Kanten oder dergleichen Unebenheiten vorgesehen, die das Reinigen erschweren würden.

Um eine Reinigung zu erleichtern, können jedoch auch Überzüge, z. B. aus Kunststoff, an der außen an der Branche ausgebildeten Schalteinrichtung 110 vorgesehen sein, so dass Kriechwege und insbesondere auch die Verschmutzung der Schalteinrichtung 110 selbst vermieden werden.

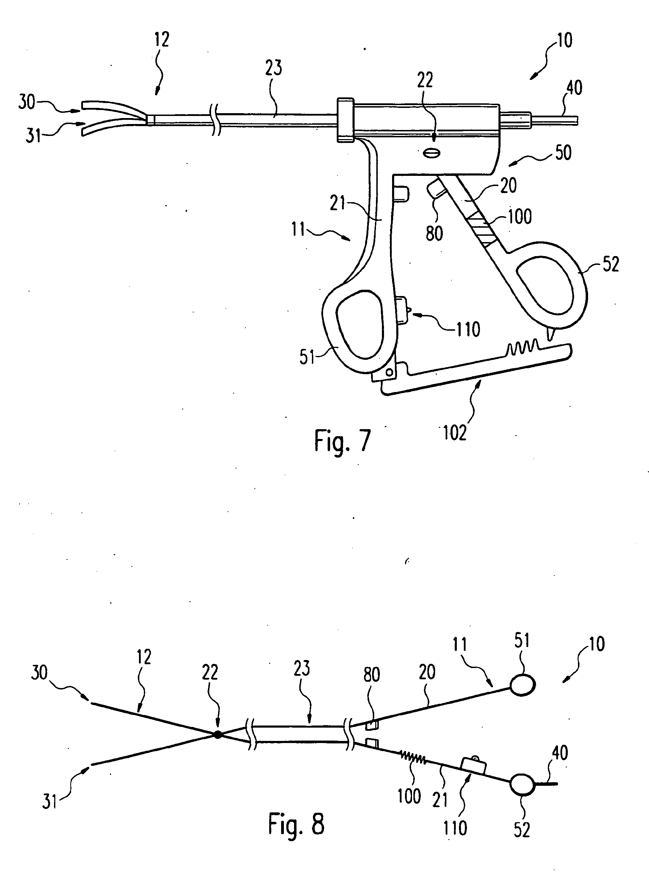

Die Fig. 7 und 8 zeigen weitere Ausführungsformen des erfindungsgemäßen Instruments, wobei die dargestellten Instrumente für die Endoskopie vorgesehen sind. Fig. 7 zeigt ein laparoskopisches Instrument 10 mit Branchen 20, 21, die an dem proximalen Bereich 11 an einem Griffelement 50 - ähnlich wie bei den oben beschriebenen Instrumenten - scherenähnlich betätigbar sind. In der Praxis sind die Branchen 20, 21 im proximalen Bereich 11 meist derart ausgebildet, dass nur eine Branche 20 bewegbar ist, während die andere Branche 21 z. B. integral mit dem Griffelement 50 ausgebildet und daher fixiert ist. Oftmals werden hier auch pistolenförmige Handgriffe angewendet. An dem distalen Bereich 12 des Instruments 10 sind die Elektrodenteile 30, 31 zum Fassen von Gewebe und zum Durchleiten des hochfrequenten Stroms durch das Gewebe ausgebildet. Die Branchen 20, 21 sind hier über einen in einem Rohrschaft 23 ausgebildeten Umlenkmechanismus (nicht gezeigt) verlängert, so dass die Bewegung der Branchen 20, 21 im proximalen Bereich 11 auf die Elektrodenteile 30, 31 übertragbar ist. Zur Betätigung der Branchen 20, 21 und damit der Elektrodenteile 30, 31 sind an dem proximalen Bereich 11 der Branchen 20, 21 die Griffeinrichtungen 51, 52 vorgesehen. Der Rohrschaft 23 ist zwischen dem proximalen Bereich 11 und dem distalen Bereich 12 der Branchen 20, 21 ausgebildet und ermöglicht das Einbringen der Elektrodenteile 30, 31 in eine Körperhöhle, wobei die Betätigung der Elektrodenteile 30, 31 von "außen" über die Griffeinrichtungen 51, 52 durchführbar ist. Beabstandungselement 80, elastisch verformbares Element 100 und Schalteinrichtung 110 sind bei dieser Ausführungsform im proximalen Bereich 11 der Branchen, im Prinzip an den Griffeinrichtungen angeordnet, so dass die Betätigung wie bei einem Schereninstrument für die offene Chirurgie durchführbar ist. Hier sei auf die Beschreibung gemäß Fig. 1 verwiesen. Die Stromzuführungseinrichtung 40 ermöglicht das Anschließen des Instruments an das elektrochirurgische Gerät. Eine Rasteinrichtung 102 - hier z. B. eine Ratsche - ermöglicht bei Bedarf das Halten der Elektrodenteile 30, 31 in ihrer Arbeitsposition (geschlossenen Position), wobei ein Überwinden der Rasteinrichtung 102, d. h. eine über die Rasteinrichtung hinausgehende fortgesetzte Bewegung der Griffeinrichtung 52, dann die Betätigung der Schalteinrichtung 110 zulässt. Grundsätzlich lassen sich die Instrumente auch derart gestalten, dass die Arbeits- oder Ruheposition auch ohne zusätzliche Einrichtung beibehalten werden kann.

Fig. 8 zeigt eine vereinfachte Darstellung eines laparoskopischen Instruments 10. Die Abbildung verdeutlicht, dass die Branchen 20, 21 zum Betätigen der Elektrodenteile 30, 31 über den im Inneren des Instruments 10 angeordneten Mechanismus (nicht gezeigt) verlängert werden, so dass minimal-invasive Eingriffe durchführbar sind. Eine Aktivierung des hochfrequenten Stromes über Beabstandungselement 80, elastisch verformbares Element 100 und Schalteinrichtung 110 erfolgt aufgrund desselben Prinzips, wie bereits mit Fig. 1 beschrieben.

Es sei erwähnt, dass auch laparoskopische Instrument mit weiteren Kombinationen von Schalteinrichtung, Beabstandungselement und elastisch verformbarem Element ausbildbar sind. So kann beispielsweise das Beabstandungselement 80 auch im distalen Bereich 12 des Instruments 10 angeordnet sein, ggf. sogar auf bzw. an den Elektrodenflächen. Das Beabstandungselement könnte dann beispielsweise gleichzeitig als Schneidabschnitt zum elektrochirurgischen Schneiden vorgesehen sein. Auch lässt sich der Minimalabstand der Elektrodenteile und damit auch der Abstand zwischen den Branchen im proximalen Bereich derart festlegen, dass die bewegbare Branche bzw. Griffeinrichtung nur bis zu einem bestimmten Abstand auf die fixierte Branche zubewegt werden kann. Eine weitere Auslenkung der Branche bzw. Griffeinrichtung über den ersten Wegbereich wäre dann nicht vorgesehen, so dass in diesem Falle auf ein explizites, außen am Instrument angeordnetes Beabstandungselement verzichtet werden könnte. Das Beabstandungelement würde dann durch eine Einschränkung der Verfahrbewegung der Branche realisiert sein. Im Übrigen sei auf die Beschreibung gemäß der Fig. 1 bis 6 verwiesen.

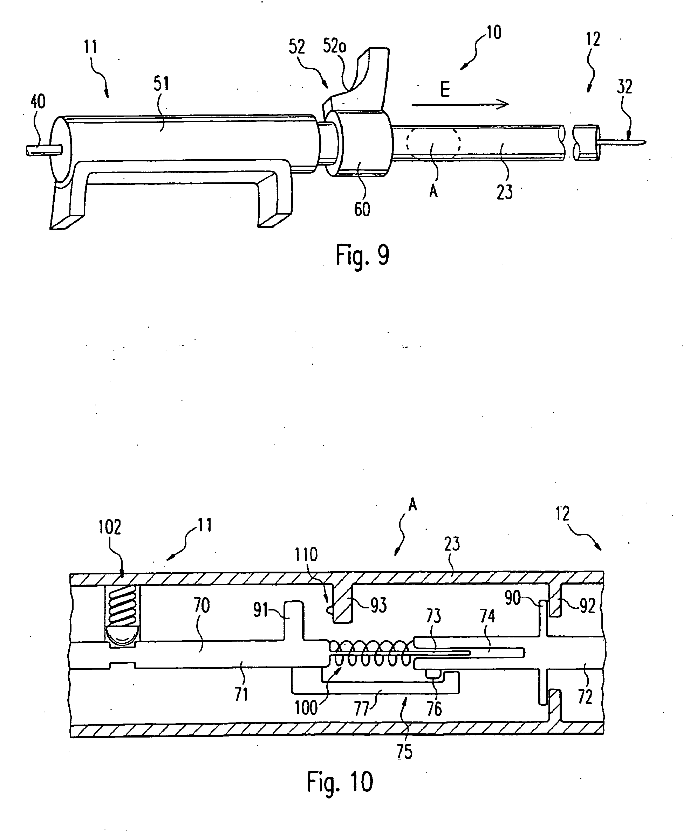

Fig. 9 zeigt eine weitere Ausführungsform des erfindungsgemäßen Instruments 10. Das Instrument ist als ein Rohrschaftinstrument, vorzugsweise für die monopolare Technik ausgebildet und kann z. B. mit einer Nadelelektrode 32 eingesetzt werden. Das Instrument 10 weist einen Schaft 23 an einem distalen Ende 12 des Instruments 10 und daran angeordnete Griffeinrichtungen 51, 52 an einem proximalen Ende 11 auf. Innerhalb des Schaftes 23 ist ein Trägerelement (hier nicht gezeigt) zum Tragen des Elektrodenteils 32 und zum Bewegen des Elektrodenteils 32 in Erstreckungsrichtung E des Schaftes (also linear) von der Ruheposition in die Arbeitsposition angeordnet. Eine detaillierte Ansicht des Trägerelements 70 ist in Fig. 10 als Detailansicht A dargestellt. Das Elektrodenteil 32 ist in der Ruheposition in den Schaft 23 zurückgezogen und damit geschützt vor äußeren Einflüssen untergebracht. Für die Behandlung von Gewebe wird das Elektrodenteil 32 aus dem Schaft 23 "herausgefahren" und kann so an dem Gewebe platziert werden. Die Bewegung des Trägerelements 70 erfolgt über ein entlang dem Schaft 23 verfahrbares Schubelement 60. Eine erste Griffeinrichtung 51, ein Handgriff, ist zum Halten des Instruments 10 vorgesehen, während eine zweite Griffeinrichtung 52 mit dem Schubelement 60 verbunden und zum Ansetzen eines Fingers, vorzugsweise des Daumens ausgebildet ist. Das Schubelement 60 lässt sich also über das Daumenelement 52a verschieben, um so das Trägerelement 70 zu bewegen. Auf diese Weise kann das Elektrodenteil 32 auf einfachste Art von der Ruhe- in die Arbeitsposition verschoben werden, wobei das Schubelement 60 mit dem Daumenelement 52a einen ersten Wegbereich durchläuft.

Fig. 10 zeigt als Detailansicht A einen Ausschnitt aus dem Trägerelement 70 in Seitenansicht, wie es in dem oben beschriebenen Schaft 23 gemäß Fig. 9 angeordnet sein kann. Das Trägerelement 70 ist gemäß Fig. 10 aus zwei Abschnitten ausgebildet, einem distalen Abschnitt 72 und einem proximalen Abschnitt 71. An dem distalen Abschnitt 72 des Trägerelements 70 ist das Elektrodenteil 32 (hier nicht gezeigt) angeordnet, um mit dem Trägerelement 70 z. B. über das Schubelement 60 bewegt zu werden. Das Trägerelement 70 weist an seinem distalen Abschnitt 72 eine erste Anschlageinrichtung 90 auf, die aus dem Trägerelement 70 hervorsteht und mit einem ersten Schaftvorsprung 92 derart zusammenwirkt, dass durch das Zusammenwirken die Arbeitsposition des Elektrodenteils 32 festlegbar ist. Das heißt, aufgrund einer Bewegung des Trägerelements 70 in Richtung des distalen Endes 12 des Instruments 10 schlägt die erste Anschlageinrichtung 90 an dem ersten Schaftvorprung 92 an und verhindert somit eine weitere Bewegung des Trägerelements 70. Anschlageinrichtung 90 und Schaftvorsprung 92 sind derart zueinander angeordnet, dass der Anschlag erfolgt, sobald sich das Elektrodenteil 32 in seiner Arbeitsposition befindet.

Das Trägerelement 70 ist nun derart ausgebildet, dass durch dessen Bewegung über die zweite Griffeinrichtung 52 eine Schalteinrichtung 110 betätigbar ist. Wie Fig. 10 zeigt, sind der distale Abschnitt 72 des Trägerelements 70 und der proximale Abschnitt 71 über ein elastisch verformbares Element 100, hier eine Schraubenfeder miteinander verbunden. Bei dieser Ausführungsform ist an dem proximalen Abschnitt 71 ein Stangenelement 73 als Verlängerung des proximalen Abschnitts 71 angeordnet, der in eine gegenüberliegende Aussparung 74 des distalen Abschnitts 72 eingreift. Das Stangenelement 73 trägt das elastisch verformbare Element 100 und bildet zusammen mit diesem die Verbindung von distalem und proximalem Abschnitt 72, 71 aus. Die Aussparung 74 des distalen Abschnitts 72 ist derart ausgelegt, dass sich das Stangenelement 73 und damit der proximale Abschnitt 71 weiter auf den distalen Abschnitt 72 zubewegen können, auch wenn sich die erste Anschlageinrichtung 90 am distalen Abschnitt 72 bereits im Anschlag an dem ersten Schaftvorsprung 92 befindet und eine Weiterbewegung des distalen Abschnittes 72 somit verhindert wird. Das Einschieben des Stangenelements 73 in die Aussparung 74 wird jedoch erst ermöglicht, wenn auf das elastisch verformbare Element 100 eine hinreichend große Kraft einwirkt, so dass dessen Verformung - z. B. eine Stauchung - die Weiterbewegung des proximalen Abschnitts 71 zulässt. Um die Weiterbewegung des proximalen Abschnitts 71 in Erstreckungsrichtung E zu ermöglichen, muss der Anwender also lediglich das Schubelement 60 in Richtung des distalen Endes 12 des Instruments 10 weiter verfahren, d. h., die Bewegung zur Überführung des Elektrodenteils 32 von der Ruhe- in die Arbeitsposition fortsetzen, wobei das Schubelement 60 mit dem Daumenelement 52a einen zweiten Wegbereich durchläuft. Die Krafteinwirkung wird erreicht, indem die erste Anschlageinrichtung 90 mit dem ersten Schaftvorsprung 92 zusammenwirkt und gleichzeitig das Schubelement 60 über den zweiten Wegbereich hinweg weiterbewegt wird. Das elastisch verformbare Element 100 dient u. a. dazu, eine Steifigkeit zwischen distalem und proximalem Abschnitt 72, 71 herzustellen, so dass die Bewegung beider Abschnitte während des Übergangs von der Ruheposition des Elektrodenteils 32 in die Arbeitsposition im Gleichlauf erfolgen kann, bis die erste Anschlageinrichtung 90 mit dem ersten Schaftvorsprung 92 zusammenwirkt. Das elastisch verformbare Element 100 schiebt also den distalen Abschnitt 72 mit in Richtung des distalen Endes 12 des Instruments 10, wenn das Trägerelement 70 über das Schubelement 60 bewegt wird.

Der proximale Abschnitt 71 weist eine zweite Anschlageinrichtung 91 auf, die mit einem zweiten Schaftvorsprung 93 zusammenwirkt, wobei die zweite Anschlageinrichtung 91 und der zweite Schaftvorsprung 93 derart angeordnet sind, dass das Zusammenwirken erst erfolgt, nachdem sich die erste Anschlageinrichtung 90 mit dem ersten Schaftvorsprung 92 im Anschlag befindet. Der zweite Schaftvorsprung 93 weist in diesem Ausführungsbeispiel die Schalteinrichtung 110 auf, die durch das Zusammenwirken von zweiter Anschlageinrichtung 91 mit dem zweiten Schaftvorsprung 93 betätigbar ist. Das heißt, durch das Hineinschieben des Stangenelements 73 am proximalen Abschnitt 71 des Trägerelements 70 in die Aussparung 74 des distalen Abschnitts 72 und durch die Stauchung des elastisch verformbaren Elements 100 lässt sich die Schalteinrichtung 110 betätigen und die Stromzufuhr aktivieren.

Um das Elektrodenteil 32 oder auch die Elektrodenteile 30, 31 eines Maulteils in ihrer Arbeitsposition zu halten, kann bei Bedarf eine Rasteinrichtung 102 (diese erzeugt im Prinzip dieselbe Wirkung wie die Rasteinrichtung gemäß Fig. 7) vorgesehen sein. Hier greift ein über eine Feder mit dem Rohrschaft gekoppelter Rastknopf in eine Ringnut an dem Trägerelement 70 ein. Der Rastknopf wird dabei in Rastknopfführungen geführt (die Bauteile der Rasteinrichtung sind nicht näher spezifiziert). Bei Schubbewegung des Trägerelements 70 in Erstreckungsrichtung E des Schaftes rastet der Rastknopf in die Ringnut ein und fixiert die Elektrode in ihrer entsprechenden Position. Das Überwinden der Einrastung (Erhöhung der Krafteinwirkung auf das Trägerelement) ermöglicht die Betätigung der Schalteinrichtung 110, wie oben beschrieben. Soll die Stromzufuhr unterbrochen werden, würde die Rückstellkraft des elastisch verformbaren Elements 100 wieder ein Einrasten der Rasteinrichtung 102 bewirken. Gegebenenfalls müsste der Anwender eine weitere Kraft aufwenden, um das Elektrodenteil oder die Elektrodenteile wieder in ihre endgültige Ruheposition zu überführen. Das elastisch verformbare Element 100 kann aber auch derart ausgebildet sein, dass dessen Rückstellwirkung zumindest zusätzlich die Überführung in die Ruheposition erleichtert. Die Schalteinrichtung 110 ist vorzugsweise als Taster 110a ausgebildet, wobei eine Deaktivierung der Stromzufuhr dann durch das "Loslassen", also dem Beabstanden der zweiten Anschlageinrichtung 91 von der Schalteinrichtung 110 erfolgt. Das heißt, die Krafteinwirkung des Anwenders auf das Schubelement wird reduziert oder ggf. völlig zurückgenommen. Ist das elastisch verformbare Element 100 nicht derart ausgebildet, dass es das Überführen in die Ruheposition unterstützt, verbleibt das Elektrodenteil bzw. verbleiben die Elektrodenteile in ihrer Arbeitsposition. Gegebenenfalls kann auch eine weitere Schalteinrichtung zur Deaktivierung der Stromzufuhr vorgesehen werden oder aber das Instrument ist derart ausgebildet, dass eine erneute Betätigung der Schalteinrichtung eine Deaktivierung bewirkt.

Grundsätzlich ließe sich oben beschriebener Mechanismus zur Betätigung der Schalteinrichtung 110 auch ohne Stangenelement 73 mit lediglicher Anordnung des elastisch verformbaren Elements 100 zwischen den Abschnitten 72, 71 des Trägerelements 70 realisieren. Das Stangenelement 73 dient hier jedoch der Stabilisierung des Trägerelements 70 zwischen den beiden Abschnitten 72, 71 und gewährleistet eine sichere Handhabung des Geräts. Ferner wird durch die Länge der Aussparung 74 festgelegt, bis zu welchem Maß das Federelement stauchbar ist.

Die Schaftvorsprünge 92, 93 können z. B. innerhalb des Schaftes 23 angeordnet, ggf. integral mit diesem ausgebildet sein und aus diesem in das Innere des Schaftes 23 hinein hervorstehen. So wirken die Vorsprünge 92, 93 als Stoppelemente, sobald die Anschlageinrichtungen 90, 91 die Schaftvorsprünge 92, 93 erreicht haben. Gleichzeitig trägt der zweite Schaftvorsprung 93 die Schalteinrichtung 110. Die Vorsprünge 92, 93 können als Einzelelemente vorgesehen sein, oder aber als eine Vielzahl von Elementen, die das jeweilige Stoppelement ausbilden. Auch können die Vorsprünge 92, 93 als vollständig umlaufende Elemente, z. B. Scheibenelemente, vorgesehen sein und so eine Art gleichförmige Verengung des Schaftes 23 ausbilden. Gleiches gilt im Prinzip für die an dem Trägerelement 70 angeordneten Anschlageinrichtungen 90, 91. Eine Anschlageinrichtung kann aus einem Einzelelement oder aus einer Vielzahl dieser Elemente bestehen.

Um ein "Einfahren" des Elektrodenteils 32 in den Schaft 23 zu gewährleisten, d. h., das Elektrodenteil 32 von der Arbeitsposition wieder in die Ruheposition zu überführen, ist beispielsweise eine Rückhalteeinrichtung 75 vorgesehen, die die beiden Abschnitte 71, 72 des Trägerelements 70 während des Einfahrens zusammenhält. Bei dem Ausführungsbeispiel gemäß Fig. 10 greift ein am proximalen Abschnitt 71 des Trägerelements 70 angeordneter Hebel 77 hinter einen am distalen Abschnitt 72 angeordneten Vorsprung 76 ein, so dass der proximale Abschnitt 71 den distalen Abschnitt 72 beim Einfahren des Elektrodenteils 32 mitnimmt. Ohne Rückhalteeinrichtung 75 würde das elastisch verformbare Element 100, sofern es an beiden Abschnitten 71, 72 befestigt ist, beim Zurückholen des Elektrodenteils 32 gedehnt werden, so dass das Überführen des Elektrodenteils 32 von der Arbeitsposition in die Ruheposition ggf. unkoordiniert erfolgen würde.

Die in Fig. 9 gezeigte ovale, gestrichelte Linie am Schaft 23 des Instruments 10 deutet an, dass der soeben beschriebene Mechanismus für die Bewegung des Trägerelements 70 zur Betätigung der Schalteinrichtung 110 gemäß Fig. 10 dort innerhalb des Schaftes 23 angeordnet sein kann. Fig. 10 zeigt dann die entsprechende Detailansicht A.

Die Bewegung des Trägerelements 70 über das Schubelement 60 setzt eine Verbindung des Trägerelements 70 mit dem Schubelement 60 voraus. So könnte das Schubelement 60 beispielsweise durch Schlitze im Schaft 23 hindurch mit dem Trägerelement 70 verbunden sein und so dessen Bewegung gewährleisten.

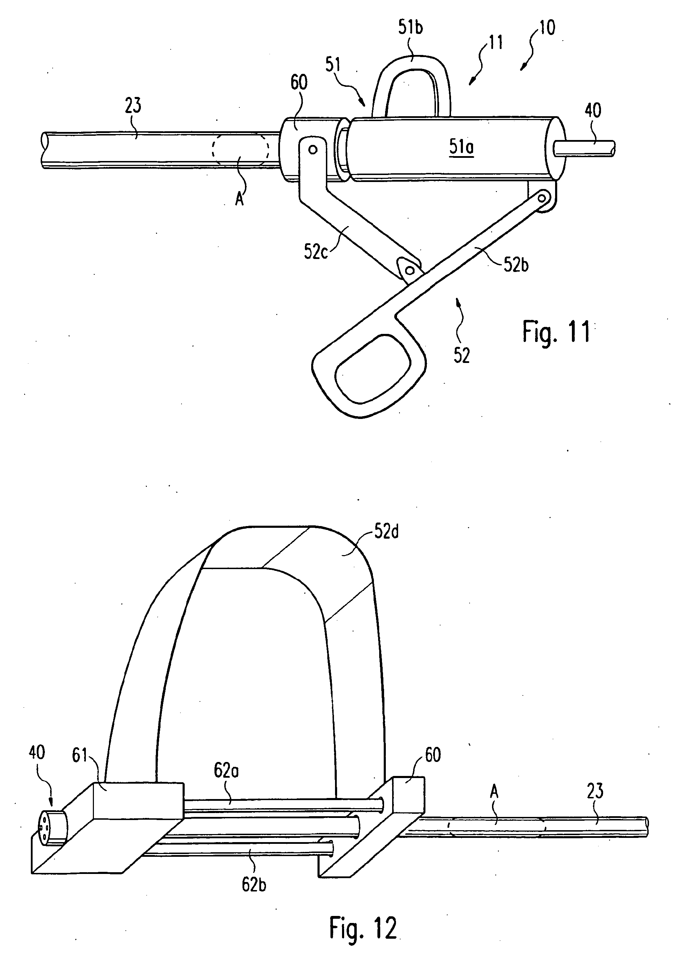

Fig. 11 zeigt einen Ausschnitt aus einem erfindungsgemäßen elektrochirurgischen Instrument 10, nämlich einen proximalen Bereich 11 des Instruments 10 in perspektivischer Ansicht. Auch hier ist ein Schubelement 60 zur Betätigung des Trägerelements (hier nicht gezeigt) vorgesehen. Die ovale, gestrichelte Linie verweist auch hier auf die Detailansicht A gemäß Fig. 10 und deutet an, dass innerhalb des Schaftes 23 der Mechanismus für die Bewegung des Trägerelements 70 zur Betätigung der Schalteinrichtung 110 gemäß Fig. 10 vorgesehen ist. Das Schubelement 60 ist hier über ein Branchenelement 52b als die zweite Griffeinrichtung 52 bewegbar, das an einem ersten Ende an der ersten Griffeinrichtung 51, also dem Handgriff des Schaftes 23 bewegbar angeordnet, also angelenkt und an einem zweiten Ende mit dem Schubelement 60 über ein bewegbares Hebelelement 52c verbunden ist. Das heißt, das bewegbare Hebelelement 52c ist sowohl am Schubelement 60, als auch am Branchenelement 52b angelenkt. Ferner ist an dem Handgriff 51a ein Bügel 51 b angebracht, um das Branchenelement 52b ähnlich einer Schere bewegen zu können. Sobald nun das Branchenelement 52b in Richtung des Schaftes 32 bewegt wird, lässt sich das Schubelement 60 entlang dem Schaft verfahren und betätigt dabei das Trägerelement 70 in der oben beschriebenen Weise. Das Durchlaufen des ersten Wegbereichs des Branchenelements 52b, also der zweiten Griffeinrichtung, verfährt das Elektrodenteil 32 in die Arbeitsposition, während das Durchlaufen des anschließenden zweiten Wegbereichs die Betätigung der Schalteinrichtung 110 und die Aktivierung des Stromes bewirkt.

Fig. 12 zeigt einen Ausschnitt aus einem erfindungsgemäßen elektrochirurgischen Instrument 10, nämlich einen proximalen Bereich 11 des Instruments 10 in perspektivischer Ansicht. Auch hier deutet die ovale, gestrichelte Linie die Detailansicht A gemäß Fig. 10 an, d. h., dass innerhalb des Schaftes 23 der Mechanismus für die Bewegung des Trägerelements 70 zur Betätigung der Schalteinrichtung 110 gemäß Fig. 10 vorgesehen ist. Das Schubelement 60 ist hier über ein federelastisches Element 52d (das ist im Prinzip der Griff) mit einem die Stromzuführungseinrichtung 40 aufweisenden Steg 61 verbunden, so dass sich das Schubelement 60 auf den Steg 61 zubewegen lässt, sobald das federelastische Element 52d zusammengedrückt wird. Führungselemente 62a und 62b stabilisieren die Schubbewegung des Trägerelements.

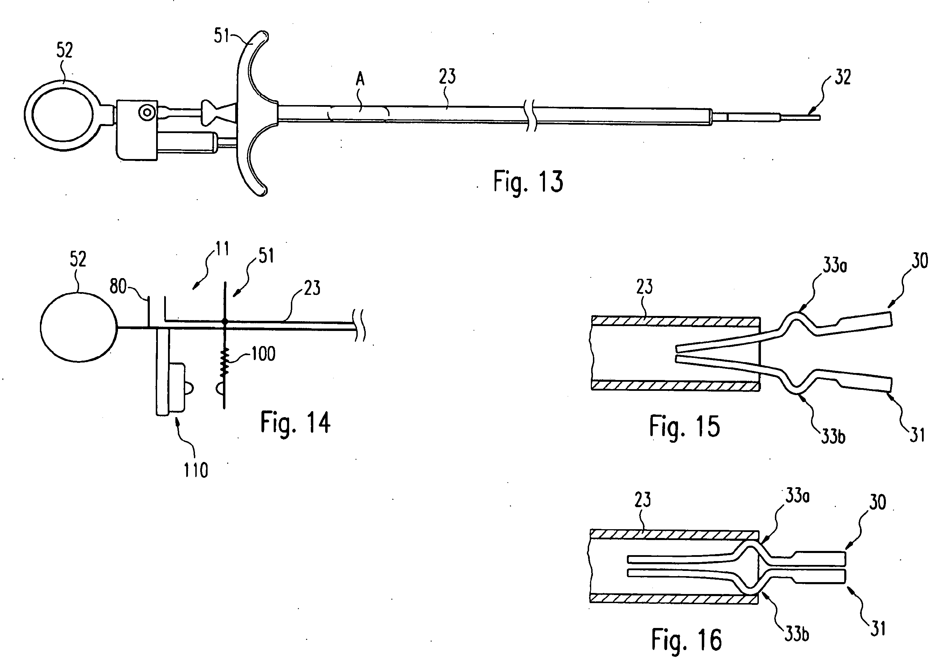

Der Handgriff gemäß Fig. 12 ist insbesondere dazu ausgebildet, eine bipolare Anordnung zu betätigen. So ist beispielsweise ein aus zwei Elektrodenteilen bestehendes Maulteil (nicht gezeigt) geöffnet (Fig. 15), während sich Schubelement 60 und federelastisches Element 52d in einer Ruheposition befinden. Durch Betätigen des Handgriffes, also dem Zubewegen des Schubelements 60 an den Steg erfolgt ein Schließen des Maulteils (Fig. 16), wobei durch das Bewegen auch die Schalteinrichtung aktiviert werden kann, so wie es z. B. gemäß Fig. 11 beschrieben ist. Wie aus Fig. 15 zu entnehmen ist, ist also das Maulteil, d. h. sind die Elektrodenteile 30, 31 geöffnet (Ruheposition). Die Betätigung des Handgriffes zieht die Elektrodenteile 30, 31 in den Rohrschaft 23 zurück, wobei sich das Maulteil schließt, d. h., die Elektrodenteile 30, 31 bewegen sich aufeinander zu. Das Schließen der Elektrodenteile 30, 31 - wie Fig. 16 zeigt - wird über die beiden Wölbungsbereiche 33a und 33b unterstützt, die unmittelbar hinter den Elektrodenteilen derart angeordnet sind, wobei deren Kontakt mit dem Inneren des Rohrschaftes das Schließen des Maulteils bewirkt. Die Wölbungsbereiche 33a, 33b sind in der Regel durch die Krümmung der Stromzuführungseinrichtungen ausgebildet. Da hier die Arbeitsposition durch das Einfahren der Elektrodenteile erreicht wird, ist die Anordnung gemäß Fig. 10 hier vorzugsweise umgekehrt, also in entgegengesetzter Richtung eingebaut, wie dies beispielsweise bei Fig. 9 vorgesehen ist. Somit lässt sich bei einer weiteren Heranführung des Schubelements 60 an den Steg 61 auch die Schalteinrichtung erfindungsgemäß betätigen.

Vorzugsweise kann das Maulteil, also können die Elektrodenteile zum Fassen des Gewebes nur so lange geschlossen bleiben, wie im Prinzip Kraft über die Griffeinrichtungen auf die Elektrodenteile ausgeübt wird. Dies ist vor allem bei schnellem Arbeiten von Vorteil. Hier muss das federelastische Element 52d als in seiner zusammengedrückten Position behalten werden, damit die Elektrodenteile 30, 31 geschlossen bleiben. Durch das "Loslassen" öffnet sich das Maulteil und es erfolgt auch - bei entsprechender Positionierung des Schubelements 60 - die Deaktivierung der Stromzufuhr.

Gegebenenfalls werden die Elektrodenteile bei Griffeinrichtungen ohne federelastischem Element (dies ist nicht zu verwechseln mit dem elastisch verformbaren Element) durch einen Rückstellmechanismus, z. B. durch eine Feder, wieder in ihre geöffnete Position gezwungen, sofern die Krafteinwirkung auf die Griffeinrichtungen reduziert wird. Ferner kann die Rasteinrichtung die geschlossenen Elektrodenteile fixieren. Gleiches gilt für die übrigen Ausführungsformen, auch für monopolare Anordnungen. Aktives Kraftaufwenden oder ein Rastmechanismus können die Beibehaltung der Arbeitsposition und/oder auch der Stromzufuhr ermöglichen.

Grundsätzlich ist es also möglich, das Elektrodenteil bzw. die Elektrodenteile (also sowohl bei monopolaren als auch bei bipolaren Anordnungen) in ihre Arbeitsposition zu bringen, wobei das Verrasten die Beibehaltung der Arbeitsposition ermöglicht. Trotz Rastmechanismus ist es dann noch möglich, die Schalteinrichtung - wie oben beschrieben - zu betätigen. Ein "Loslassen" bzw. eine Kraftreduzierung über die Griffeinrichtungen könnte dann zwar die Stromzufuhr unterbrechen, trotzdem würde aber die Arbeitsposition beibehalten werden. Möglich ist es auch, das Instrument derart auszubilden, dass mit der Deaktivierung der Stromzufuhr auch das Elektrodenteil bzw. die Elektrodenteile wieder in ihre Ruheposition bzw. in die geöffnete Position übergehen. Die Instrumente können jedoch auch ohne Rastmechanismus ausgebildet sein, so dass die entsprechenden Ruhe- bzw. Arbeitspositionen durch aktive Betätigung der Griffeinrichtungen erreicht werden.

Fig. 13 zeigt eine weitere Ausführungsform eines erfindungsgemäßen elektrochirurgischen Instruments 10. Die ovale, gestrichelte Linie deutet auch hier die Detailansicht A gemäß Fig. 10 an, d. h., dass innerhalb des Schaftes 23 der Mechanismus für die Bewegung des Trägerelements 70 zur Betätigung der Schalteinrichtung 110 gemäß Fig. 10 vorgesehen ist. Das Gegeneinanderbewegen der Griffeinrichtungen 51, 52 erfolgt hier ähnlich der Betätigung einer Injektionsspritze, wobei die ringförmige zweite Griffeinrichtung 52 gegenläufig zur ersten Griffeinrichtung 51 bewegt wird, um so das Elektrodenteil 32 in die Arbeitsposition zu verfahren und anschließend die Schalteinrichtung 110 zu aktivieren. Als Elektrode 32 kommt hier beispielsweise eine Nadelelektrode für monopolares Koagulieren und/oder Schneiden in Betracht, die über das Trägerelement 70 im Inneren des Rohrschaftes 23 bewegt wird. Das fortschreitende Gegeneinanderbewegen der Griffeinrichtungen ermöglicht die Betätigung der Schalteinrichtung, wie bereits oben beschrieben.

Das in Fig. 14 gezeigte elektrochirurgische Instrument 10 entspricht im Wesentlichen dem in Fig. 13 gezeigten. Die vereinfachte Darstellung gibt einen proximalen Bereich 11 des Instruments 10 in Seitenansicht wieder, allerdings ist hier die Schalteinrichtung 110 außerhalb des Instruments 10 angeordnet und kann durch Biegung oder Knickung beispielsweise der ersten Griffeinrichtung 51 bei bereits zusammengeführten Griffeinrichtungen betätigt werden. Hierzu ist das elastisch verformbare Element 100, z. B. ein Biegeelement - wie bereits oben näher erläutert (vgl. z. B. Fig. 1) - an der Griffeinrichtung 51 angeordnet, um diese in mit der Griffeinrichtung 52 zusammengeführtem Zustand in Richtung der Schalteinrichtung 110 weiter zu bewegen. Damit lässt sich die Schalteinrichtung betätigen. Ein Beabstandungselement 80 (Anschlag) definiert den ersten Wegbereich beim Zusammenführen der Griffeinrichtungen, während das elastisch verformbare Element das Durchlaufen des zweiten Wegbereichs ermöglicht.

Bei pistolenförmigen Handgriffen, wie sie oft bei laparoskopischen Instrumenten vorgesehen werden, lässt sich z. B. eine linear bewegbare Griffeinrichtung zur Betätigung des Trägerelements auf den Pistolenhandgriff zubewegen und durchläuft dabei den ersten Wegbereich. Ein mit der bewegbaren Griffeinrichtung gekoppeltes, z. B. vorgespanntes Federelement ließe auch bei dieser Ausführungsform das Durchlaufen des zweiten Wegbereichs zur Betätigung der Schalteinrichtung zu.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Proximaler Bereich, proximales Ende
- 12: Distaler Bereich, distales Ende
- 20: Branche
- 21: Branche
- 22: Gelenk
- 23: Rohrschaft, Schaft
- 30: Elektrodenteil
- 31: Elektrodenteil
- 32: Elektrodenteil
- 33a: Wölbungsbereich
- 33b: Wölbungsbereich
- 40: Stromzuführungseinrichtung
- 50: Griffelement
- 51: (Erste) Griffeinrichtung
- 52: (Zweite) Griffeinrichtung
- 51a: Handgriff
- 51b: Bügel ,
- 52a: Daumenelement
- 52b: Branchenelement
- 52c: Hebelelement
- 52d: Federelastisches Element
- 60: Schubelement
- 61: Steg
- 62a: Führungselement
- 62b: Führungselement
- 70: Trägerelement
- 71: Proximaler Abschnitt
- 72: Distaler Abschnitt
- 73: Stangenelement
- 74: Aussparung
- 75: Rückhalteeinrichtung
- 76: Vorsprung
- 77: Hebel
- 80: Beabstandungselement
- 90: Erste Anschlageinrichtung
- 91: Zweite Anschlageinrichtung
- 92: Erster Schaftvorsprung
- 93: Zweiter Schaftvorsprung
- 100: Elastisch verformbares Element
- 101: Elastisch verformbares Element
- 102: Rasteinrichtung
- 110: Schalteinrichtung
- 110a: Taster
- 110b: Reedkontakt
- 111: Abdeckungselement
- 112: Öffnungsbereich
- 113: Betätigungselement: Betätigungszapfen, Magnetelement

- A: Detailansicht gemäß Fig. 10
- E: Erstreckungsrichtung

## Patentansprüche

1. Elektrochirurgisches Instrument (10) zum Koagulieren und/oder Schneiden biologischen Gewebes, umfassend
- zwei gegeneinander bewegbar verbundene Branchen (20, 21),
- Griffeinrichtungen (51, 52) an einem proximalen Bereich (11) der Branchen (20, 21) bzw. des Instruments (10) zum Zusammenführen der Branchen (20, 21),
- Elektrodenteile (30, 31) an einem distalen Bereich (12) der Branchen (20, 21) bzw. des Instruments (10) zum Fassen von Gewebe und zum Durchleiten eines HF-Stromes durch das Gewebe,
- Stromzuführungseinrichtungen (40) zum Zuführen des HF-Stromes zu den Elektrodenteilen (30, 31) von einem HF-Generator,
- eine Schalteinrichtung (110) zum Aktivieren des HF-Stromes bei zusammengeführten Branchen (20, 21)
- mindestens ein Beabstandungselement (80) zur Ausbildung eines definierten Minimalabstandes zwischen den Elektrodenteilen (30, 31),
- mindestens ein elastisch verformbares Element (100), das derart an mindestens einer der Branchen (20, 21) oder den Griffeinrichtungen (51, 52) angeordnet ist, dass beim Schließen der Branchen (20, 21) und Erreichen des Minimalabstandes mindestens ein Bereich der Griffeinrichtungen (51, 52) im proximalen Bereich (11) zur Betätigung der Schalteinrichtung (110) weiter bewegbar ist,
**dadurch gekennzeichnet, dass**
- das elastisch verformbare Element (100) an der Branche (20, 21) oder an mindestens einer der Griffeinrichtungen (51, 52), diese durchsetzend angeordnet und derart ausgebildet ist, dass durch das elastisch verformbare Element (100) eine Soll-Biege- oder -Knickstelle an der Branche (20, 21)oder der Griffeinrichtung (51, 52) vorgesehen ist.

2. Elektrochirurgisches Instrument nach Anspruch 1,
**dadurch gekennzeichnet, dass**
das elastisch verformbare Element (100) zwischen der Schalteinrichtung (110) und dem Beabstandungselement (80) im proximalen Bereich (11) der Branchen (20, 21) angeordnet ist.

3. Elektrochirurgisches Instrument nach einem der vorhergehenden Ansprüche,
**dadurch gekennzeichnet, dass**
das elastisch verformbare Element (100) als ein Branchenabschnitt oder ein Griffeinrichtungsabschnitt vorgesehen ist, der gegenüber umgebenden Branchenbereichen oder Griffeinrichtungsbereichen verjüngt ausgebildet ist, so dass durch den Branchenabschnitt oder den Griffeinrichtungsabschnitt eine Soll-Biege - oder -Knickstelle an der Branche (20, 21) oder der Griffeinrichtung (51, 52) vorgesehen ist.

4. Elektrochirurgisches Instrument nach einem der vorhergehenden Ansprüche,
**dadurch gekennzeichnet, dass**
das Beabstandungselement (80) als Anschlagelement an mindestens einer Branche (20, 21) ausgebildet ist.

5. Elektrochirurgisches Instrument nach einem der vorhergehenden Ansprüche,
**dadurch gekennzeichnet, dass**
die Schalteinrichtung (110) und die gegenüberliegende Branche (20, 21) oder Griffeinrichtung (51, 52) derart ausgebildet sind, dass die Schalteinrichtung (110) durch Aufsetzen der gegenüberliegenden Branche (20, 21) oder Griffeinrichtung (51, 52) betätigbar ist.

6. Elektrochirurgisches Instrument nach einem der vorhergehenden Ansprüche,
**dadurch gekennzeichnet, dass**
ein an der der Schalteinrichtung (110) gegenüberliegenden Branche (20, 21) oder Griffeinrichtung (51, 52) ausgebildetes Betätigungselement (113) vorgesehen ist, das derart angeordnet ist, dass es die Schalteinrichtung (110) bei Weiterbewegung des mindestens einen Bereichs der Griffeinrichtungen (51, 52) betätigt.

7. Elektrochirurgisches Instrument nach einem der vorhergehenden Ansprüche,
**dadurch gekennzeichnet, dass**
ein die Schalteinrichtung (110) umgebendes Abdeckungselement (111) mit einem in Richtung des Betätigungselements (113) ausgebildeten Öffnungsbereich (112) vorgesehen ist,
wobei das Betätigungselement (113) die Schalteinrichtung (110) durch den Öffnungsbereich (112) hindurch betätigt.

8. Elektrochirurgisches Instrument nach einem der vorhergehenden Ansprüche,
**dadurch gekennzeichnet, dass**
die Schalteinrichtung (110) als Reedkontakt (110b) ausgebildet ist,
wobei ein Magnetelement (113) zur Betätigung des Reedkontaktes (110b) an der Branche (20, 21) oder der Griffeinrichtung (51, 52) angeordnet ist, die dem Reedkontakt (110b) gegenüberliegt.

9. Elektrochirurgisches Instrument (10) zum Koagulieren und/oder Schneiden biologischen Gewebes, umfassend
- einen Schaft (23),
- mindestens eine erste Griffeinrichtung (51) und eine zweite Griffeinrichtung (52) an einem proximalen Bereich (11) des Instruments (10) zu dessen Handhabung, wobei die Griffeinrichtungen (51, 52) gegeneinander bewegbar sind,
- mindestens ein Elektrodenteil (32) an einem distalen Bereich (12) des Instruments (10) zum Durchleiten eines HF-Stromes durch das Gewebe, wobei das Elektrodenteil (32) mittels der Griffeinrichtungen (51, 52) von einer Ruheposition in eine Arbeitsposition bringbar ist,
- Stromzuführungseinrichtungen (40) zum Zuführen des HF-Stromes zu dem mindestens einen Elektrodenteil (32) von einem HF-Generator,
- eine Schalteinrichtung (110) zum Aktivieren des HF-Stromes bei zusammengeführten Griffeinrichtungen (51, 52),
- mindestens ein elastisch verformbares Element (100), das derart an und/oder in dem Instrument (10) angeordnet ist, dass mindestens ein Bereich der Griffeinrichtungen (51, 52) zur Betätigung der Schalteinrichtung (110) weiter bewegbar ist, wenn sich das Elektrodenteil (32) in der Arbeitsposition befindet, **dadurch gekennzeichnet, dass**
- das elastisch verformbare Element (100) an mindestens einer Griffeinrichtung (51, 52), diese durchsetzend angeordnet und derart ausgebildet ist, dass durch das elastisch verformbare Element (100) eine Soll-Biege- oder -Knickstelle an der Griffeinrichtung vorgesehen ist.

10. Elektrochirurgisches Instrument nach Anspruch 9,
**dadurch gekennzeichnet, dass**
innerhalb des Schaftes (23) ein über die Griffeinrichtungen (51, 52) bewegbares Trägerelement (70) zum Tragen des mindestens einen Elektrodenteils (32) und zum Bewegen des Elektrodenteils (32) in Erstreckungsrichtung (E) des Schaftes (23) von der Ruheposition in die Arbeitsposition angeordnet ist.

11. Elektrochirurgisches Instrument nach Anspruch 9 oder 10,
**dadurch gekennzeichnet, dass**
ein bei einem Zusammenführen der Griffeinrichtungen (51, 52) wirksam werdendes Beabstandungselement (80) derart in oder an dem Instrument (10) vorgesehen ist, dass durch dieses die Arbeitsposition des Elektrodenteils (32) festlegbar ist.

12. Elektrochirurgisches Instrument nach einem der Ansprüche 9 bis 11,
**dadurch gekennzeichnet, dass**
zwei Elektrodenteile (30, 31) an dem Instrument vorgesehen sind, die über das Trägerelement (70) in Erstreckungsrichtung (E) des Schaftes (23) von der Ruheposition in die Arbeitsposition bewegbar sind.

13. Elektrochirurgisches Instrument nach einem der Ansprüche 9 bis 12,
**dadurch gekennzeichnet, dass**
das elastisch verformbare Element (100) als ein Griffeinrichtungsabschnitt vorgesehen ist, der gegenüber umgebenden Griffeinrichtungsbereichen verjüngt ausgebildet ist, so dass durch den Griffeinrichtungsabschnitt eine Soll-Biege- oder - Knickstelle an der Griffeinrichtung (51, 52) vorgesehen ist.

14. Elektrochirurgisches Instrument nach einem der Ansprüche 9 bis 13,
**dadurch gekennzeichnet, dass**
die Schalteinrichtung (110) als Reedkontakt (110b) ausgebildet ist,
wobei ein Magnetelement (113) zur Betätigung des Reedkontaktes (110b) an mindestens einer Griffeinrichtung (51, 52) angeordnet ist.

15. Elektrochirurgisches Instrument nach einem der vorhergehenden Ansprüche,
**dadurch gekennzeichnet, dass**
die Schalteinrichtung (110) als Schalter, Taster oder dergleichen Element ausgebildet ist.

16. Elektrochirurgisches Instrument nach einem der vorhergehenden Ansprüche,
**dadurch gekennzeichnet, dass**
das Instrument (10) für die offene Chirurgie oder für die Endoskopie ausgebildet ist.

17. Elektrochirurgisches Instrument nach einem der vorhergehenden Ansprüche,
**dadurch gekennzeichnet, dass**
das elastisch verformbare Element (100) aus einem Polyetheretherketon oder dergleichen Kunststoff oder aus einem Federstahl ausgebildet ist.

## Claims

1. Electrosurgical instrument (10) for coagulating and/or cutting biological tissue, comprising
- two connected branches (20, 21) movable towards each other,
- gripping devices (51, 52) at a proximal area (11) of the branches (20, 21), or of the instrument (10), for bringing the branches (20, 21) together,
- electrode parts (30, 31) at a distal area (12) of the branches (20, 21), or of the instrument (10), for grasping tissue and for conducting an HF current through the tissue,
- current supply devices (40) for supplying the HF current to the electrode parts (30, 31) from an HF generator,
- a switching device (110) for activating the HF current when the branches (20, 21) are brought together,
- at least one spacing element (80) for forming a defined minimum spacing between the electrode parts (30, 31),
- at least one elastically deformable element (100), which is arranged on at least one of the branches (20, 21) or the gripping devices (51, 52) in such a way that, when the branches (20, 21) are closed and reach the minimum spacing, at least one area of the gripping devices (51, 52) can be moved further in the proximal area (11) in order to actuate the switching device (110),
**characterized in that**
- the elastically deformable element (100) is arranged on the branch (20, 21) or on at least one of the gripping devices (51, 52), passing through the latter, and is designed such that a predetermined bending or buckling point on the branch (20, 21) or the gripping device (51, 52) is provided by the elastically deformable element (100).

2. Electrosurgical instrument according to Claim 1, **characterized in that** the elastically deformable element (100) is arranged between the switching device (110) and the spacing element (80) in the proximal area (11) of the branches (20, 21).

3. Electrosurgical instrument according to one of the preceding claims, **characterized in that** the elastically deformable element (100) is provided as a branch section or gripping-device section that is narrower than surrounding branch areas or gripping-device areas, such that a predetermined bending or buckling point on the branch (20, 21) or the gripping device (51, 52) is provided by the branch section or the gripping-device section.

4. Electrosurgical instrument according to one of the preceding claims, **characterized in that** the spacing element (80) is designed as a stop element on at least one branch (20, 21).

5. Electrosurgical instrument according to one of the preceding claims, **characterized in that** the switching device (110) and the opposite branch (20 21) or gripping device (51, 52) are designed in such a way that the switching device (110) can be actuated by contact with the opposite branch (20, 21) or gripping device (51, 52).

6. Electrosurgical instrument according to one of the preceding claims, **characterized in that** an actuating element (113) is formed on the branch (20, 21) or gripping device (51, 52) opposite the switching device (110) and is arranged in such a way that it actuates the switching device (110) upon further movement of the at least one area of the gripping devices (51, 52).

7. Electrosurgical instrument according to one of the preceding claims, **characterized in that** a cover element (111) is provided, which surrounds the switching device (110) and has an opening area (112) formed in the direction of the actuating element (113), wherein the actuating element (113) actuates the switching device (110) through the opening area (112).

8. Electrosurgical instrument according to one of the preceding claims, **characterized in that** the switching device (110) is designed as a reed contact (110b), wherein a magnet element (113) for actuating the reed contact (110b) is arranged on the branch (20, 21) or gripping device (51, 52) lying opposite the reed contact (110b).

9. Electrosurgical instrument (10) for coagulating and/or cutting biological tissue, comprising:
- a shaft (23),
- at least a first gripping device (51) and a second gripping device (52) at a proximal area (11) of the instrument (10) for handling the latter, wherein the gripping devices (51, 52) are movable towards each other,
- at least one electrode part (32) at a distal area (12) of the instrument (10), for conducting an HF current through the tissue, wherein the electrode part (32) can be brought from a rest position to a working position by means of the gripping devices (51, 52),
- current supply devices (40) for supplying the HF current to the at least one electrode part (32) from an HF generator,
- a switching device (110) for activating the HF current when the gripping devices (51, 52) are brought together,
- at least one elastically deformable element (100), which is arranged on and/or in the instrument (10) in such a way that at least one area of the gripping devices (51, 52) can be moved further in order to actuate the switching device (110) when the electrode part (32) is in the working position,
**characterized in that**
- the elastically deformable element (100) is arranged on at least one gripping device (51, 52), passing through the latter, and is designed in such a way that a predetermined bending or buckling point on the gripping device is provided by the elastically deformable element (100).

10. Electrosurgical instrument according to Claim 9, **characterized in that** a support element (70) is arranged inside the shaft (23), which support element (70) is movable via the gripping devices (51, 52), supports the at least one electrode part (32) and moves the electrode part (32), in the direction of extension (E) of the shaft (23), from the rest position to the working position.

11. Electrosurgical instrument according to Claim 9 or 10, **characterized in that** a spacing element (80), which becomes active when the gripping devices (51, 52) are brought together, is provided in or on the instrument (10) in such a way as to define the working position of the electrode part (32).

12. Electrosurgical instrument according to one of Claims 9 to 11, **characterized in that** two electrode parts (30, 31) are provided on the instrument and, by way of the support element (70), are movable in the direction of extension (E) of the shaft (23) from the rest position to the working position.

13. Electrosurgical instrument according to one of Claims 9 to 12, **characterized in that** the elastically deformable element (100) is provided as a gripping-device section that is narrower than surrounding gripping-device areas, such that a predetermined bending or buckling point on the gripping device (51, 52) is provided by the gripping-device section.

14. Electrosurgical instrument according to one of Claims 9 to 13, **characterized in that** the switching device (110) is designed as a reed contact (110b), wherein a magnet element (113) for actuating the reed contact (110b) is arranged on at least one gripping device (51, 52).

15. Electrosurgical instrument according to one of the preceding claims, **characterized in that** the switching device (110) is designed as a switch, button or similar element.

16. Electrosurgical instrument according to one of the preceding claims, **characterized in that** the instrument (10) is designed for open surgery or for endoscopy.

17. Electrosurgical instrument according to one of the preceding claims, **characterized in that** the elastically deformable element (100) is made from a polyetheretherketone or similar plastic or from a spring steel.

## Revendications

1. Instrument électro-chirurgical (10) destiné à coaguler et/ou couper des tissus biologiques, comprenant :
- deux branches reliées de manière à ce qu'elles soient mobiles l'une par rapport à l'autre (20, 21),
- des dispositifs formant poignées (51, 52) dans une région proximale (11) des branches (20, 21) ou de l'instrument (10) destinés à réunir les branches (20, 21),
- des parties d'électrodes (30, 31) dans une région distale (12) des branches (20, 21) ou de l'instrument (10) destinées à saisir un tissu et à faire passer un courant HF à travers le tissu,
- des dispositifs d'alimentation en courant (40) destinés à faire en sorte qu'un générateur HF alimente en courant HF les parties d'électrodes (30, 31),
- un dispositif de commutation (110) destiné à activer le courant HF dans les branches réunies (20, 21),
- au moins un élément d'espacement (80) pour établir une distance minimale définie entre les parties d'électrodes (30, 31),
- au moins un élément déformable élastiquement (100) qui est disposé sur au moins l'une des branches (20, 21) ou des dispositifs formant poignées (51, 52) de telle manière que lors de la fermeture des branches (20, 21) et lorsque la distance minimale est atteinte, au moins une région des dispositifs formant poignées (51, 52) puisse être déplacée davantage dans la région proximale (11) pour actionner le dispositif de commutation (110), **caractérisé en ce que**
l'élément déformable élastiquement (100) est disposé sur la branche (20, 21) ou sur au moins l'un des dispositifs formant poignées (51, 52) en sollicitant ces derniers et est conçu de manière à définir un point de flexion ou de gauchissement souhaité sur la branche (20, 21) ou sur le dispositif formant poignée (51, 52) au moyen de l'élément déformable élastiquement (100).

2. Instrument électro-chirurgical selon la revendication 1, **caractérisé en ce que** l'élément déformable élastiquement (100) est disposé entre le dispositif de commutation (110) et l'élément d'espacement (80) dans la région proximale (11) des branches (20, 21).

3. Instrument électro-chirurgical selon l'une quelconque des revendications précédentes, **caractérisé en ce que** l'élément déformable élastiquement (100) est prévu en tant que partie de branche ou que partie de dispositif formant poignée, laquelle partie est réalisée de manière à être biseautée vers les régions avoisinantes des branches ou des dispositifs formant poignées, de manière à définir un point de flexion ou de gauchissement souhaité sur la branche (20, 21) ou sur le dispositif formant poignée (51, 52) au moyen de la partie de branche ou de la partie de dispositif formant poignée.

4. Instrument électro-chirurgical selon l'une quelconque des revendications précédentes, **caractérisé en ce que** l'élément d'espacement (80) est réalisé sous la forme d'un élément de butée sur au moins une branche (20, 21).

5. Instrument électro-chirurgical selon l'une quelconque des revendications précédentes, **caractérisé en ce que** le dispositif de commutation (110) et la branche (20, 21) ou le dispositif formant poignée (51, 52) opposé(e) sont réalisés de telle manière que le dispositif de commutation (110) puisse être actionné par mise en place de la branche (20, 21) ou du dispositif formant poignée (51, 52) opposé(e).

6. Instrument électro-chirurgical selon l'une quelconque des revendications précédentes, **caractérisé en ce qu'**il est prévu un élément d'actionnement (113) sur la branche (20, 21) ou sur le dispositif formant poignée (51, 52) opposé(e) au dispositif de commutation (110), lequel élément est disposé de manière à actionner le dispositif de commutation (110) lors d'un mouvement supplémentaire de l'au moins une région des dispositifs formant poignées (51, 52).

7. Instrument électro-chirurgical selon l'une quelconque des revendications précédentes, **caractérisé en ce qu'**il est prévu un élément formant couvercle (111) entourant le dispositif de commutation (110) comprenant une région d'ouverture (112) formée dans la direction de l'élément d'actionnement (113), dans lequel l'élément d'actionnement (113) actionne le dispositif de commutation (110) au moyen de la région d'ouverture (112).

8. Instrument électro-chirurgical selon l'une quelconque des revendications précédentes, **caractérisé en ce que** le dispositif de commutation (110) est réalisé sous la forme d'un contact à lames souples (110b), dans lequel un élément magnétique (113) destiné à actionner le contact à lames souples (110b) est disposé sur la branche (20, 21) ou sur le dispositif formant poignée (51, 52), lequel élément est opposé au contact à lames souples (110b).

9. Instrument électro-chirurgical (10) destiné à coaguler et/ou couper des tissus biologiques, comprenant :
- un arbre (23),
- au moins un premier dispositif formant poignée (51) et un second dispositif formant poignée (52) dans une région proximale (11) de l'instrument (10) destiné à sa manipulation, dans lequel les dispositifs formant poignées (51, 52) sont mobiles l'un par rapport à l'autre,
- au moins une partie d'électrode (32) dans une région distale (12) de l'instrument (10), destinée à faire passer un courant HF à travers le tissu, dans lequel la partie d'électrode (32) peut être amenée à passer d'une position de repos à une position de fonctionnement au moyen des dispositifs formant poignées (51, 52),
- des dispositifs d'alimentation en courant (40) destinés à faire en sorte qu'un générateur HF alimente en courant HF l'au moins une partie d'électrode (32),
- un dispositif de commutation (110) destiné à activer le courant HF lorsque les dispositifs formant poignées (51, 52) sont réunis,
- au moins un élément déformable élastiquement (100) qui est disposé sur et/ou dans l'instrument (10) de manière à ce qu'au moins une région des dispositifs formant poignées (51, 52) puisse être déplacée davantage pour actionner le dispositif de commutation (110) lorsque la partie d'électrode (32) se trouve en position de fonctionnement, **caractérisé en ce que** l'élément déformable élastiquement (100) est disposé sur au moins l'un des dispositifs formant poignées (51, 52) en sollicitant ces derniers et est conçu de manière à définir un point de flexion ou de gauchissement souhaité sur le dispositif formant poignée au moyen de l'élément déformable élastiquement (100).

10. Instrument électro-chirurgical selon la revendication 9, **caractérisé en ce qu'**il est prévu à l'intérieur de l'arbre (23) un élément de support (70) destiné à supporter l'au moins une partie d'électrode (32) et à déplacer la partie d'électrode (32) dans la direction d'extension (E) de l'arbre (23) de la position de repos à la position de fonctionnement, lequel élément de support peut être déplacé au moyen des dispositifs formant poignées (51, 52).

11. Instrument électro-chirurgical selon la revendication 9 ou 10, **caractérisé en ce qu'**un élément d'espacement (80) devenant fonctionnel lors de la réunion des dispositifs formant poignées (51, 52) est prévu dans ou sur l'instrument (10) de manière à pouvoir fixer la position de fonctionnement de la partie d'électrode (32) à l'aide de celui-ci.

12. Instrument électro-chirurgical selon l'une quelconque des revendications 9 à 11, **caractérisé en ce qu'**il est prévu sur l'instrument deux parties d'électrodes (30, 31) pouvant être déplacées au moyen de l'élément de support (70) dans la direction d'extension (E) de l'arbre (23) de la position de repos à la position de fonctionnement.

13. Instrument électro-chirurgical selon l'une quelconque des revendications 9 à 12, **caractérisé en ce que** l'élément déformable élastiquement (100) est prévu en tant que partie de dispositif formant poignée, laquelle partie est réalisée de manière biseautée vers les régions des dispositifs formant poignées avoisinantes, de telle manière qu'un point de flexion ou de gauchissement souhaité soit défini sur le dispositif formant poignée (51, 52) au moyen de la partie de dispositif formant poignée.

14. Instrument électro-chirurgical selon l'une quelconque des revendications 9 à 13, **caractérisé en ce que** le dispositif de commutation (110) est réalisé sous la forme d'un contact à lames souples (110b), dans lequel un élément magnétique (113) destiné à actionner le contact à lames souples (110b) est disposé sur au moins un dispositif formant poignée (51, 52).

15. Instrument électro-chirurgical selon l'une quelconque des revendications précédentes, **caractérisé en ce que** le dispositif de commutation (110) est réalisé sous la forme d'un commutateur, d'un bouton ou d'un autre élément de ce type.

16. Instrument électro-chirurgical selon l'une quelconque des revendications précédentes, **caractérisé en ce que** l'instrument (10) est conçu pour la chirurgie effractive ou pour l'endoscopie.

17. Instrument électro-chirurgical selon l'une quelconque des revendications précédentes, **caractérisé en ce que** l'élément déformable élastiquement (100) est réalisé à partir d'une polyéther éther cétone ou d'une matière plastique de ce type ou à partir d'un acier à ressort.
